# EUROPEAN PATENT SPECIFICATION

(11) **EP 1 252 183 B1**
(45) Date of publication and mention of the grant of the patent: **24.05.2006**
(21) Application number: 00987364.7
(22) Date of filing: 01.12.2000
(51) Int. Cl.: C07K 14/31

(54) **METHOD FOR REDUCING THE IMMUNOGENICITY OF STAPHYLOKINASE BY ELIMINATION OF T-CELL EPITOPES**
METHODE ZUR REDUZIERUNG DER IMMUNOGENIZITÄT VON STAPHYLOKINASE DURCH ENTFERNEN VON T-ZELL EPITOPEN
PROCEDE DE REDUCTION DE L'IMMUNOGENICITE DE LA STAPHYLOKINASE PAR L'ELIMINATION D'EPITOPES DE LYMPHOCYTES T

(30) Priority: 02.12.1999 EP 99204093
(43) Date of publication of application: 30.10.2002
(73) Proprietor: Thromb-X N.V., 3000 Leuven (BE)
(72) Inventor: WARMERDAM, Petronella, Adriana, Maria, NL-3010 Kessel-Lo (BE); PLAISANCE, Stéphane, Daniel, Nöel, Georges, Henri, B-3012 Wilsele (BE); COLLEN, Désiré, José, London SW5 0HN (GB); DE MAEYER, Marc, Cyriel, Hilda, B-1702 Groot-Bijgaarden (BE)
(74) Representative: Van Someren, Petronella F. H. M.
(86) International application number: PCT/EP2000/012299
(87) International publication number: WO 2001/040281

(56) References cited:
- WO-A-00/34317
- WO-A-98/52976
- WO-A-99/53038
- GUNDLACH B R ET AL: "Determination of T cell epitopes with random peptide libraries" JOURNAL OF IMMUNOLOGICAL METHODS,NL,ELSEVIER SCIENCE PUBLISHERS B.V.,AMSTERDAM, vol. 192, no. 1, 10 June 1996 (1996-06-10), pages 149-155, XP004020829 ISSN: 0022-1759
- STAUSS H J ET AL: "INDUCTION OF CYTOTOXIC T LYMPHOCYTES WITH PEPTIDES IN VITRO: IDENTIFICATION OF CANDIDATE T-CELL EPITOPES IN HUMAN PAPILLOMA VIRUS" PROCEEDINGS OF THE NATIONAL ACADEMY OF SCIENCES OF USA,US,NATIONAL ACADEMY OF SCIENCE. WASHINGTON, vol. 89, 1 September 1992 (1992-09-01), pages 7871-7875, XP002031413 ISSN: 0027-8424
- ERB K J ET AL: "Identification of potential CD8+ T-cell epitopes of the 19 kDa and AhpC proteins from Mycobacterium tuberculosis. No evidence for CD8+ T-cell priming against the identified peptides after DNA-vaccination of mice" VACCINE,GB,BUTTERWORTH SCIENTIFIC. GUILDFORD, vol. 16, no. 7, 1 April 1998 (1998-04-01), pages 692-697, XP004112257 ISSN: 0264-410X
- CHAUX PASCAL ET AL: "Identification of MAGE-3 epitopes presented by HLA-DR molecules to CD4+ T lymphocytes." JOURNAL OF EXPERIMENTAL MEDICINE, vol. 189, no. 5, 1 March 1999 (1999-03-01), pages 767-777, XP002165455 ISSN: 0022-1007
- MANICI SIMONA ET AL: "Melanoma cells present a MAGE-3 epitope to CD4+ cytotoxic T cells in association with histocompatibility leukocyte antigen DR11." JOURNAL OF EXPERIMENTAL MEDICINE, vol. 189, no. 5, 1 March 1999 (1999-03-01), pages 871-876, XP002165456 ISSN: 0022-1007
- COLLEN ET AL: "Thrombolytic properties of poorly immunogenic variants of recombinant staphylokinase" FIBRINOLYSIS AND PROTEOLYSIS,US,NEW YORK, NY, vol. 12, no. SUPPL. 01, June 1998 (1998-06), page 30 XP002111034 ISSN: 1369-0191
- COLLEN D ET AL: "Recombinant staphylokinase variants with altered immunoreactivity. I: Construction and characterization" CIRCULATION,US,AMERICAN HEART ASSOCIATION, DALLAS, TX, vol. 94, no. 2, 15 July 1996 (1996-07-15), pages 197-206, XP002111037 ISSN: 0009-7322
- COLLEN D ET AL: "Recombinant staphylokinase variants with altered immunoreactivity. II: Thrombolytic properties and antibody induction" CIRCULATION,US,AMERICAN HEART ASSOCIATION, DALLAS, TX, vol. 94, no. 2, 15 July 1996 (1996-07-15), pages 207-216, XP002111036 ISSN: 0009-7322
- COLLEN D ET AL: "Recombinant staphylokinase variants with altered immunoreactivity. III: Species variability of antibody binding patterns" CIRCULATION,US,AMERICAN HEART ASSOCIATION, DALLAS, TX, vol. 95, no. 2, 21 January 1997 (1997-01-21), pages 455-462, XP002111035 ISSN: 0009-7322
- COLLEN D ET AL: "Recombinant staphylokinase variants with altered immunoreactivity. IV: Identification of variants with reduced antibody induction but intact potency" CIRCULATION,US,AMERICAN HEART ASSOCIATION, DALLAS, TX, vol. 95, no. 2, 21 January 1997 (1997-01-21), pages 463-472, XP002111038 ISSN: 0009-7322
- LAROCHE YVES ET AL: "Recombinant staphylokinase variants with reduced antigenicity due to elimination of B-lymphocyte epitopes." BLOOD, vol. 96, no. 4, 15 August 2000 (2000-08-15), pages 1425-1432, XP002172610 ISSN: 0006-4971

## Description

The present invention relates to a method for reducing the immunogenicity of staphylokinase by elimination of T-cell epitopes. The invention furthermore relates to a method for producing staphylokinase having a reduced T-cell reactivity and to the staphylokinase variants, thus obtained. The invention also relates to the use of modified staphylokinase in therapy, diagnosis and prophylaxis.

An immune response involves, firstly, recognizing foreign material (pathogen/antigen) and secondly, mounting a reaction to eliminate it. Broadly speaking, two types of reactions can be triggered; an innate or non-specific response and an adaptive or highly antigen-specific response. It is the latter reaction that improves with each successive encounter with the same foreign material. The immune system remembers the foreign material, and deals with it more promptly, generating a life-long immunity following initial exposure.

Lymphocytes play a central role in all adaptive immune responses, since they specifically recognize individual pathogens/antigens, whether they come from inside host cells or outside in the tissue fluids or blood. In fact, there are several different types of lymphocytes, but they fall into two basic categories, T-lymphocytes (or T-cells) and B-lymphocytes (or B-cells).

B-cells combat antigens and pathogens and their products by releasing specific antibodies.

T-cells can be divided in different types and display a wider range of activities. The so-called cytotoxic T-cells are responsible for the destruction of host cells, which have become infected with viruses or other pathogens. T helper cells can interact with phagocytes and help to destruct pathogens. Other T helper cells are involved in the control of B-lymphocyte development and antibody production.

Each B-cell is genetically programmed to encode a unique surface receptor specific for a particular antigen. Having recognized its specific antigen, the B-cells can proliferate and differentiate into plasma cells, which produce large amounts of the receptor molecule in a soluble form, known as the antibody.

While B-lymphocytes recognize conformational epitopes (3-D surface structure) on native antigens, T-cells recognize linear amino acid sequences (T-cell epitopes). But they can only do so, when the T-cell epitope is presented on the cell surface of other cells by the so-called human histocompatibility leucocyte antigens (HLA) molecules. The antigen is not presented by the HLA molecules as intact proteins, but rather as processed peptides non-covalently bound in their peptide-binding groove. This processing and presentation can be done by specialized antigen presenting cells (APC), which are capable of stimulating T-cells, or by infected cells, which then become a target for cytotoxic T-cells. The T-lymphocytes can recognize these HLA-peptide complexes by virtue of a specific receptor, called the T-cell receptor (TCR). Each TCR is unique, it recognizes a specific peptide bound in the groove of a particular HLA-molecule. Upon recognition, the specific T-cell receives an activation signal, then a secondary signal is needed to induce a T-cell response, which includes proliferation and cytokine production.

If an individual encounters an antigen, and the immune system answers with a humoral response, a series of cellular and molecular interactions occur in an orderly sequence. The first interaction involves antigen-specific T-cells and APC. Its outcome largely dictates the subsequent course of events. A sufficient number of T-helper cells needs to be triggered appropriately. Specific T-cells then respond with proliferation and cytokine secretion.

Subsequently, these activated T-helper cells interact with specific B-cells that present antigen to them. This B-T-cell contact in combination with the secreted cytokines, will activate the B-cells and subsequently induce proliferation and differentiation into plasma cells. In turn, they produce antibodies, and depending on the T-cell signals they received, differentiate further by class switching, affinity maturation and the development of an immunological memory.

Thus, for secondary or subsequent antigen encounter, a larger number of antigen-specific B-cells is present, making them the major type of APC, and importantly induce a shorter circuit in the immunological response. However, if the T-helper cells are not triggered simultaneously, a form of immunological tolerance can develop, and no immunological events will follow.

Based on these considerations, the present inventors contemplated that the elimination of functional T-cell epitopes in staphylokinase will also have a great influence on the induction of a humoral response. If fewer or no specific T-cells are triggered, because specific peptides can no longer be efficiently presented, B-lymphocytes will be hampered to mature and, consequently, the antibody production will be abrogated.

In the prior art methods are known for reducing immunogenicity of therapeutic proteins by detecting and removing T-cell epitopes contained therein, see International patent applications WO 99/53038 and WO98/52976. Also is described the generation of staphylokinase variants with altered immunoreativity in which B-cell epitopes are removed, see Collen et al., "Thrombolytic properties of pooly immunogene variants of recombinant staphylokinase", Fibrimolysis and Proteolysis, US, New York, NY, vol. 12, no. SUPPL. 01, June 1998, page 30 and Collen, D et al., "Recombinant staphylokinase variants with altered immunoreactivity I: Construction and characterization", circulation, US, American Heart Association, Dallas TX, vol. 94, no. 2, 15 July 1996, pages 197 - 206.

It is thus a first object of the present invention to provide a way of reducing the T-cell based immunogenicity of staphylokinase.

This object is achieved by a method for reducing the immunogenicity of staphylokinase, which method comprises the steps of:
a) designing a series of overlapping test peptides each having an amino acid sequence that corresponds with part of the amino acid sequence of staphylokinase
b) identifying which of the test peptides of the series comprises one or more T-cell epitope(s);
c) modifying the amino acid sequence of one or more of the test peptides comprising one or more T-cell epitope(s);
d) repeating step b) and optionally step c) with the modified test peptides until one or more of the T-cell epitopes originally comprised therein are significantly reduced or eliminated;
e) modifying the amino acid sequence of staphylokinase according to the T-cell eliminating modifications made in the amino acid sequence of the test peptides to produce a modified staphylokinase
   **characterized in that** the identification of test peptides comprising a T-cell epitope is performed by a combination of the following tests:
   1) a functional T-cell assay in which proliferation of one or more T-cell clones after stimulation of the T-cell clone(s) with one of the test peptides means that the said test peptide comprises a potential T-cell epitope; and/or
   2) a functional T-cell assay in which proliferation of one or more T-lymphocytes as present in the circulation of humans (PBMC) after stimulation of the PBMC with one of the test peptides means that the said test peptide comprises a T-cell epitope; and
   3) determining the interaction energy of the said test peptide with the binding groove of one or more HLA-DR haplotypes by means of computer modeling, in which the test peptide is identified as comprising a potential T-cell epitope when according to its interaction energy it fits into the HLA-DR binding groove,
wherein a test peptide is considered to comprise a T-cell epitope if it is identified in test 1) and/or 2) and 3).

The above described method refers to the minimum amount of steps necessary. It is possible to make suitable modifications to test peptides in only one round. In case such modifications are sufficient this will appear in the repetition of step b). Such modifications can then be transferred to the peptide or protein of which the immunogenicity is to be reduced. It is however also possible to find upon repetition of step b) that further modifications are necessary. Step c) might refer also to more rounds of modification without an intermediate check for T-cell epitopes as in step b).

In addition to the above steps, there are further preferred steps that can be part of the method. It is for example possible to test the modified test peptides or the modified peptide or protein for their potential to activate T-cells and induce proliferation of T-lymphocytes as present in the circulation of humans (PBMC test). This represents an ex vivo situation and gives insight in the effect of the modifications on the in vivo immunogenicity.

Furthermore, it is possible to do the whole process of identification, modification (steps b) and c)) and optional PBMC testing all over again with the already modified peptide or protein.

The step of modifying the amino acid sequence of staphylokinase according to the T-cell eliminating modifications made in the amino acid sequence of the test peptides can for example be performed by expression of a DNA sequence encoding the modified amino acid sequence in a suitable host.

The identification of peptides comprising a T-cell epitope can be performed in various ways. In one embodiment use is made of a functional T-cell assay in which proliferation of one or more T-cell clones after stimulation of the T-cell clone(s) with one of the test peptides means that the said test peptide comprises a T-cell epitope. In a second embodiment use is made of a functional T-cell assay in which proliferation of one or more T-lymphocytes as present in the circulation of humans (PBMC) after stimulation of the PBMC with one of the test peptides means that the said test peptide comprises a T-cell epitope. In these embodiments actual peptides are used that are synthesized on the basis of the amino acid sequence of the peptide or protein of which the T-cell based immunogenicity is to be reduced.

Alternatively, the identification of peptides comprising a T-cell epitope is performed by determining the interaction energy of the test peptide with the binding groove of one or more HLA-DR haplotypes by means of computer modeling, in which test peptide(s) are identified as comprising a T-cell epitope when according to their interaction energy they fit into the HLA-DR binding groove. In this embodiment, the peptides are not actually synthesized but virtual peptides are used.

Of the test 1), 2) and 3), one test is a check for the other. Only a T-cell assay can give information on the functionality of the T-cell epitope. A peptide that theoretically fits the HLA binding groove may not lead to T-cell proliferation.

The present invention thus deals with the identification of functional human T-cell epitopes (immunogenic regions) in staphylokinase, preferably based on the combined results of computer modeling of test peptides in the HLA-DR binding groove and human T-cell analysis. More specifically, the present invention relates to a method calculating the interaction energy between two proteins/peptides.

Step d) of the above described method of the invention deals with the re-evaluation of newly introduced epitopes in the modified protein, preferably by combining the results of computer modeling of the modified test peptides in the HLA-DR binding groove and human T-lymphocyte screening with the modified protein.

The purpose of the method described above is the reduction of the immunogenicity of staphylokinase by elimination of the identified immunogenic regions. The invention according to a further aspect thereof relates to methods to remove the immunogenic regions by either deletion or insertion of the coding sequences, or by humanization of the coding sequences, or by in vitro mutagenesis of the coding sequences to replace one or more codons for wild type amino acids by a codon for another residue, or by making the immunogenic regions more sensitive for the proteolytic endosomal enzymes. The latter type of modification can influence the manner in which the T-cell epitope is presented on antigen presenting cells. In addition, modification of immunogenic regions to make them more sensitive for proteolytic enzymes can result in the generation of peptides that are too short to fit into and bind the HLA-DR groove. It is known that 9 amino acids fit in the groove, but that a minimum of 11 amino acids is necessary for binding. Changing the position of the target sites for endogenous proteolytic enzymes may lead in vivo to the production of shorter peptides that do no longer bind the HLA-DR groove.

The present invention also relates to methods for producing modified staphylokinase of the invention by preparing a DNA fragment encoding at least the part of the structural information of the peptide or protein that provides for its biological activity; mutating the DNA fragment to eliminate therefrom the coding information for one or more T-cell epitopes; cloning the mutated DNA fragment in a suitable vector, transforming or transfecting a suitable host cell with the vector, and culturing the host cell under conditions suitable for expressing the DNA fragment.

More specifically, the invention provides a method for producing a peptide or protein having a reduced immunogenicity in comparison to wild type peptide or protein, comprising the following steps:
a) providing a DNA sequence encoding at least the amino acid sequence of the peptide or protein that provides for the biological activity thereof;
b) identifying and modifying one or more of the T-cell epitopes comprised in the amino acid sequence of the wild type staphylokinase by:
   1) identifying which of the test peptides of the series comprises one or more T-cell epitope(s) by means of test 1) and/or 2) and 3) as defined in claim 1;
   2) modifying the amino acid sequence of one or more of the test peptides comprising one or more T-cell epitope(s);
   3) testing the modified peptides for their potential to activate T-cells and induce proliferation of either T-cell clones or T-lymphocytes as present in the circulation of humans (PBMC);
   4) modifying the amino acid sequence of the staphylokinase of which the immunogenicity is to be reduced according to the T-cell eliminating modifications made in the amino acid sequence of the test peptides;
   5) construction and purification of the modified staphylokinase of which the immunogenicity is to be reduced in a suitable host, and testing its biological activity;
   6) repeating step 2-5) with the modified staphylokinase until one or more of the T-cell epitopes originally comprised therein are significantly reduced or eliminated and contemplating the biological activity;
c) modifying the DNA sequence to introduce the modifications to the T-cell epitope(s) in the amino acid sequence encoded thereby;
d) introducing the DNA sequence into a host organism;
e) culturing the host organism under conditions allowing expression of the modified staphylokinase; and
f) isolating the staphylokinase having a reduced immunogenicity from the host organism.

In the above methods further steps may be incorporated as described above for the method for reducing the immunogenicity of staphylokinase.

The staphylokinase variants that can be produced according to the invention and that have a reduced T-cell based immunogenicity as compared to their wild type counterparts are novel and thus also part of this invention. The invention is applicable to reduce the T-cell based immunogenicity of all types of proteins and glycoproteins, either from human origin or originating from plants, micro-organisms or animals. Targets are also fusion proteins consisting in part of a protein from the host in which the protein is produced and in part of the staphylokinase. Other targets for the method of the invention are humanized staphylokinases.

When a protein or peptide is of human origin it can still be immunogenic when administered to a human. An example of this is the administration to a human individual of a protein for which the individual is deficient. The individual's body does not recognize the protein as "self", even though it may be of human origin, and will develop an immune response. Another example is when a protein of human origin is no longer entirely human, such as human interferon-α produced in E.coli which is not glycosylated and found to be immunogenic, or when proteins of human origin are modified to obtain better properties, such as an increased solubility. In particular, the invention is useful for lowering the T-cell based immunogenicity of the non-human protein. staphylokinase, but also for streptokinase or antibodies from other species or fragments thereof, or chimeric proteins, or fusion-proteins, or de-novo proteins for the diagnostics or treatment of human disease.

According to the invention, staphylokinase derivatives are provided that have a reduced T-cell based immunogenicity as compared to wild type staphylokinase.

While modifying a protein or peptide for reducing its immunogenicity care should be taken that the biological activity of the protein or peptide is not severely decreased or even lost. It is thus preferred that after producing a modified staphylokinase, its biological activity is tested. Tests for determining the activity of staphylokinase are usually available in the state of the art.

According to a further aspect thereof, the invention relates to the modified staphylokinase for use in treatment, diagnosis or prophylaxis and to the use of a modified staphylokinase for the preparation of a pharmaceutical composition for treatment, diagnosis or prophylaxis of a human subject.

The present invention according to a further aspect thereof relates to pharmaceutical compositions comprising at least one of the less immunogenic derivatives according to the invention together with a suitable excipient, for diagnostics or treatment in humans. Such compositions may be prepared by combining (e.g. mixing, dissolving etc.) the active compound with pharmaceutically acceptable excipients of neutral character (such as aqueous or non-aqueous solvents, stabilizers, emulsifiers, detergents, additives). The concentration of the active ingredient in a therapeutic composition may vary widely, depending on the character of the disease and the mode of administration.

The present invention relates to so-called "T-cell based immunogenicity" which means that according to the invention T-cell epitopes are identified,
characterized and modified. The invention does not relate to general methods for the identification, production and use of staphylokinase derivatives showing a reduced antigenicity as compared to wild type staphylokinase, based on the elimination of B-cell epitopes as previously described in US-5,695,754, US-5,951,980 and WO-9940198. These methods are totally distinct from and are not the subject of the present invention, although the antigenic regions (recognized by antibodies and B-cells) described in these patent publications may (partly) overlap by serendipity with immunogenic regions involved in the T-cell response (T-cell recognition).

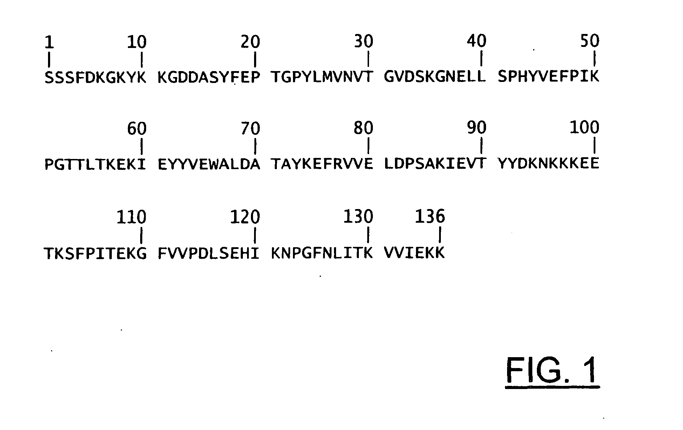

In the method of the invention, the modified protein is staphylokinase. Staphylokinase, a protein produced by certain strains of Staphylococcus aureus, which was shown to have fibrinolytic properties more than 5 decades ago appears to constitute a potent thrombolytic agent in patients with acute myocardial infarction. The staphylokinase gene has been cloned from the bacteriophages sakøC (Sako and Tsuchida, 1983) and sak42D (Behnke and Gerlach, 1987) as well as from the genomic DNA (sakSTAR) of a lysogenic Staphylococcus aureus strain (Collen et al., 1992). The staphylokinase gene encodes a protein of 163 amino acids, with amino acid 28 corresponding to the NH₂-terminal residue of full-length mature staphylokinase. The mature protein sequence of the wild type variant SakSTAR is represented in Fig. 1. Only four nucleotide differences were found in the coding regions of the sakøC, sak42D and sakSTAR genes, one of which constituted a silent mutation.

More in particular the invention relates to staphylokinase variants in which one or more of the following immunogenic regions are modified such that the T cell epitopes contained therein are eliminated:
1-SSSFDKGKYKKGDDASY-17,
16-SYFEPTGPYLMVNVTGV-32,
56-TKEKIEYYVEWALDATA-72,
71-TAYKEFRVVELDPSAKI-87,
106-ITEKGFVVPDLSEHIKN-122, and
120-IKNPGFNLITKVVIEKK-136.
excluding the variant having the combination of the following mutations: K74A, E75A, R77A, E80A, D82A.

Furthermore, the invention relates to staphylokinase variants with reduced T-cell reactivity as compared to wild type.staphylokinase, but retaining its biological activity, which variants have the amino acid sequence depicted in Figure 1 with one or more of the following amino acid substitutions in one or more of the following immunogenic region:
a) in the immunogenic region comprising SakSTAR residues 16-32 the substitutions Y17L; F18L; F18E; E19D; P20Y, Y24A; P20Y, Y24S; G22S; G22P, P23G; N28S;
b) in immunogenic region comprising SakSTAR residues 71-82 the substitutions F76W, V79Y, D82A; R77A, E80A; R77S, E80S; R77A, E80A, D82A; K74Q, R77S, E80S; K74Q, R77S, E80S, D82G; K74Q, R77S, E80S, D82A; K74Q, R77S, E80A, D82A; K74Q, R77A, E80S, D82A; K74Q, R77S, E80S, D82S; V79Y, D82A;
c) in immunogenic region comprising SakSTAR residues 106-122 the substitutions V112T; V112S; D115N; E118S; H119A; H119S; V89L, L11GY; V89L, L116T; V112T, H119S; V112T, H119A; V112T, E118S, H119S;
d) in immunogenic region comprising SakSTAR residues 120-136, the substitution K130Y.

It is preferred that such staphylokinase variants have a combination of one or more of the mutations listed above, in particular one of the following combination of mutations V112T, H119S, K130Y; R77E, E134R; V29L, L127V; K74Q, R77E, E80S, D82S, E134R; R77S, E80S, V112T, H119S; R77S, E80S, V112T, H119S, K130Y; R77A, E80A, V112T, H119S; R77A, E80A, V112T, H119S, K130Y; K74Q, R77S, E80S, V112T, H119S; K74Q, R77S, E80S, V112T, H119S, K130Y; K74Q, R77S, E80S, D82S, V112T, H119S; K74Q, R77S, E80S, D82S, V112T, H119S, K130Y.

Preferred staphylokinase variants are selected from the group consisting of SakSTAR(P20Y, Y24A), SakSTAR(P20Y, Y24S), SakSTAR(E19D), SakSTAR(Y17L), SakSTAR(F18L), SakSTAR(F18E), SakSTAR(G22S), SakSTAR(G22S, P23G), SakSTAR(N28S), and SakSTAR(V29L, L127V), SakSTAR(R77E, E134R), SakSTAR(K74Q, R77E, E80S, D82S, E134R), SakSTAR(K74Q, R77E, E80S, D82S,V112T, H119S, E134R), SakSTAR(K74Q, R77E, E80S, D82S,V112T, H119S, E134R, K130Y); SakSTAR(R77S, E80S, V112T, H119S), sakSTAR(R77S, E80S, V112T, H119S, K130Y), SakSTAR(R77A, E80A, V112T, H119S), SakSTAR(R77A, E80A, V112T, H119S, K130Y), sakSTAR(K74Q, R77S, E80S, V112T, H119S), SakSTAR(K74Q, R77S, E80S, V112T, H119S, K130Y), sakSTAR(K74Q, R77S, E80S, D82S, V112T, H119S), SakSTAR(K74Q, R77S, E80S, D82S, V112T, H119S, K130Y), SakSTAR(V112T, H119A), SakSTAR(V112T, H119S), SakSTAR(V112T, E118S, H119S), SakSTAR(V112T, H119S, K130Y), SakSTAR(V112T), SakSTAR(V112S), SakSTAR(H119A), SakSTAR(H119S), SakSTAR(V89L, L116Y), SakSTAR(V89L, L116T), SakSTAR(V112T), SakSTAR(V112S), SakSTAR(D115N), SakSTAR(E118S), SakSTAR(K130Y).

It was found that a staphylokinase variant in which at least residue K130 of the wild type amino acid sequence is replaced by a Y is particularly preferred.

To test whether the variants produced still retain their thrombolytic activity a test can be used as described in Collen et al., (1996b).

The staphylokinase variants can be used in treatment, diagnosis or prophylaxis. The invention furthermore relates to the use of a staphylokinase variant for the preparation of a pharmaceutical composition for treatment, diagnosis or prophylaxis of a human subject and to a pharmaceutical composition comprising a staphylokinase variant together with a pharmaceutically acceptable carrier, diluents or excipient.

The invention will be further elucidated in the examples that follow, using staphylokinase as a model of an immunogenic non-human protein with therapeutic potential, but this example is not intended to limit the scope of the invention, since several alternative applications will be obvious for the persons skilled in the art.

In the examples reference is made to the following figures:
**Figure 1**. Primary sequence of staphylokinase (SakSTAR). The amino acids are given in the one letter code: A, alanine; D, aspartic acid; E, glutamic acid; F, phenylalanine; G, glycine; H, histidine; I, isoleucine; K, lysine; L, leucine; M, methionine; N, asparagine; P, proline; Q, glutamine; R, arginine; S, serine; T, threonine; V, valine; W, tryptophane; and Y, tyrosine.

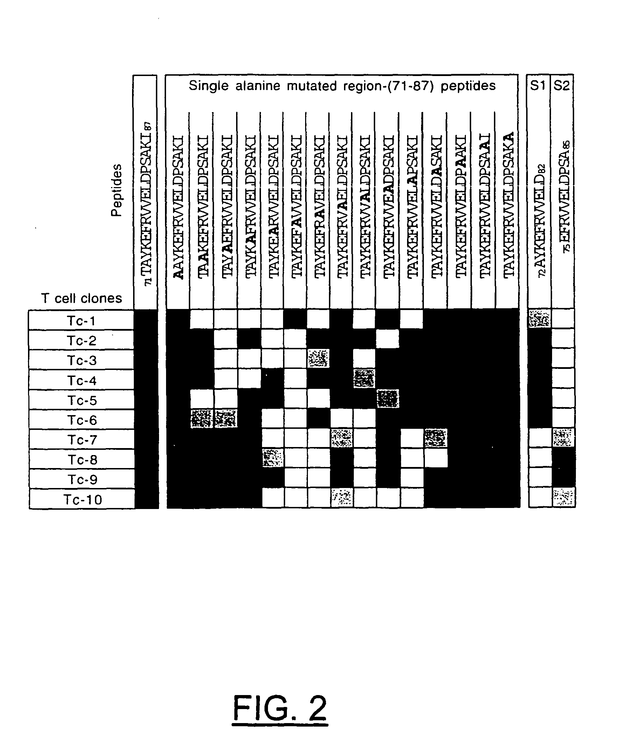

**Figure 2**. Minimal epitopes analysis of a representative panel of region(71-87)-specific T-cell clones. The T-cell clones were cultured with antigen presenting cells and SakSTAR peptide 71-TAYKEFRVVELDPSAKI-87, 15 peptides with single alanine substitutions in the region 71-87, and two 11-mer peptides (S1, S2), as indicated. If the proliferation of a T-cell clone with the corresponding peptide was found comparable to that induced by the wild-type peptide (first column), a dark fill is plotted. A light gray fill indicates a significantly lower proliferation value compared to that with the same concentration of the SakSTAR-(71-87) peptide, whereas a white box represents no detectable proliferation for the combination.

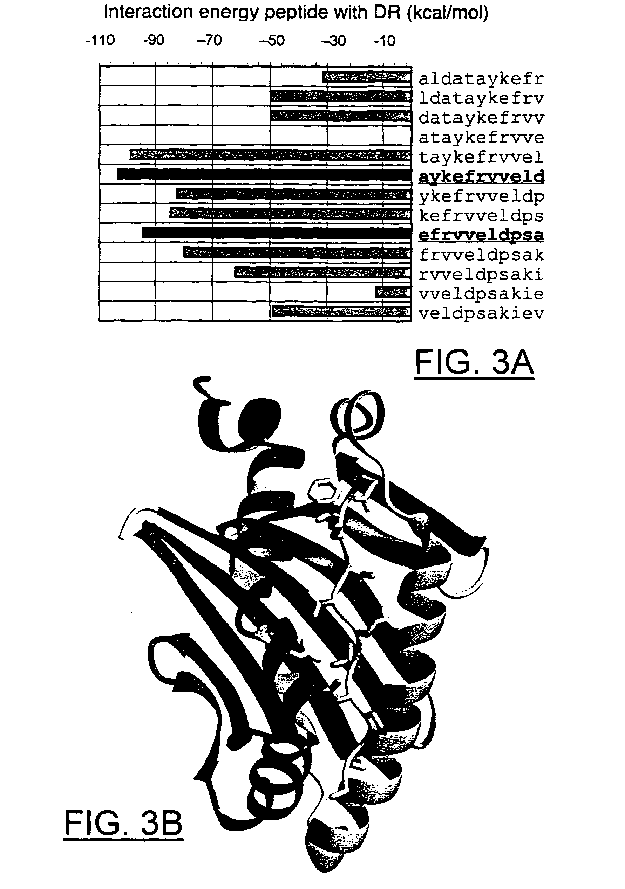

**Figure 3**. a: Modeling of 13 peptides (11-mer) encompassing the immunogenic region in SakSTAR. The non-bonded interaction energy (kcal/mol) of each peptide with the HLA-DR1 structure is plotted. Longer bars denote a stronger HLA-DR1 binding. The two selected peptides, S1 and S2, are shown in black bars. **b**: Modeled structure of the representative peptide S2 in ribbon representation (Koradi et al., 1996). The S2 peptide is shown in light gray, HLA-DR1 α- and β-chain are color coded respectively in middle and dark gray. The side chain of β-chain R71 interacting with peptide residue D82 is highlighted.

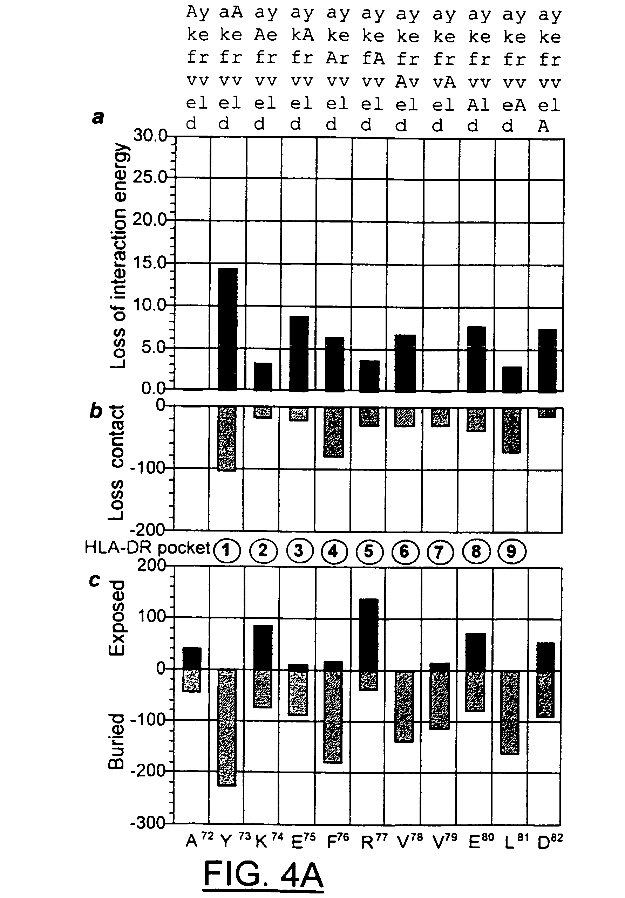

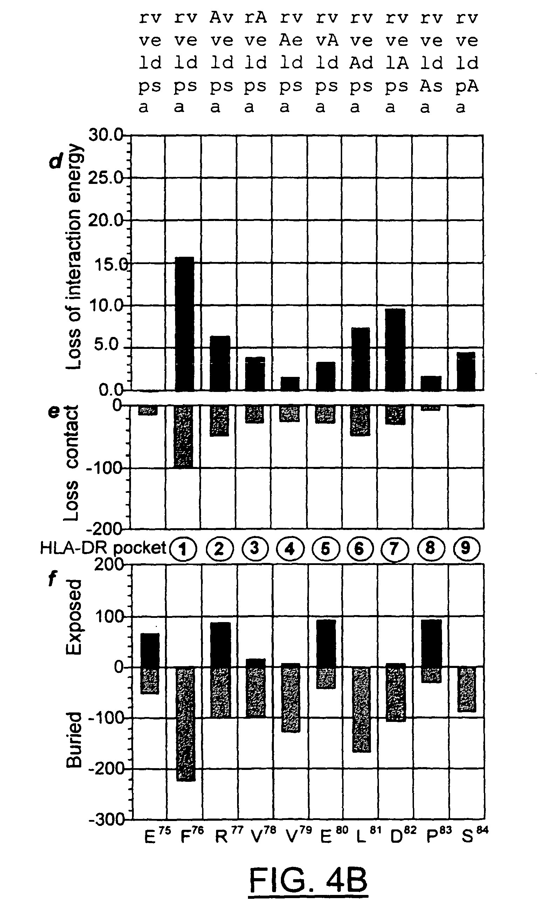

**Figure 4**. Alanine scanning experiment of the S1 (**a,b,c**) and S2 (**d,e,f**) peptide in the context of HLA-DR7. a,d: The loss of interaction energy (kcal/mol) for each alanine mutant as compared to the wild type peptide is plotted. Longer bars mean that the corresponding peptide binds less well in the peptide-binding groove. **b,e**: Bar graph of the loss of contact area (Å²) for the same series of peptides relative to the wild-type SakSTAR peptide. **c,f:** Plot of the solvent-accessible and buried surface area for each residue of the peptide bound in the groove. Positive values represent side chains exposed to the solvent in the context of the complex, which are potentially available for T-cell recognition. Negative values indicate the extent of burying in the corresponding HLA-DR pockets, numbered 1 to 9.

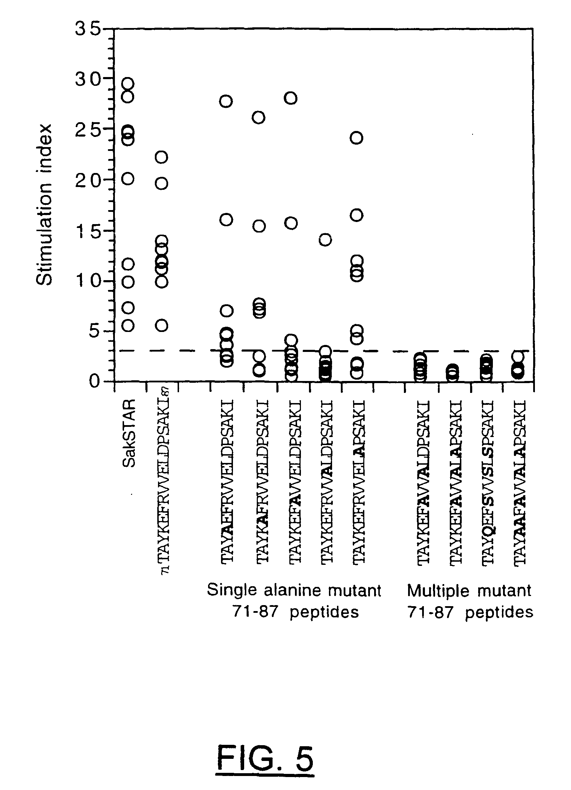

**Figure 5**. Proliferation responses of staphylokinase (SakSTAR-myc)-primed PBMC to SakSTAR-myc, the SakSTAR peptide 71-87 or peptide derivatives thereof. PBMC from ten region-(71-87)-immunoreactive donors were primed for SakSTAR-myc and re-stimulated with irradiated CD3-depleted autologous PBMC and with no antigen, SakSTAR-myc, wild type SakSTAR(71-87) peptide TAYKEFRVVELDPSAKI, five single mutation(71-87) peptides (TAY**A**EFRVVELDPSAKI, TAYK**A**FRVVELDPSAKI, TAYKEF**A**VVELDPSAKI, TAYKEFRVV**A**LDPSAKI, TAYKEFRVVEL**A**PSAKI), and four multiple mutation(71-87) peptides (TAYKEF**A**VV**A**LDPSAKI, TAYKEF**A**VV**A**L**A**PSAKI, TAY**Q**EFSVVSLSPSAKI, TAY**AA**F**A**VV**A**L**A**PSAKI). The cellular response is plotted as stimulation index. Each individual spot represents a cellular response from a single donor to the indicated antigens/peptides.

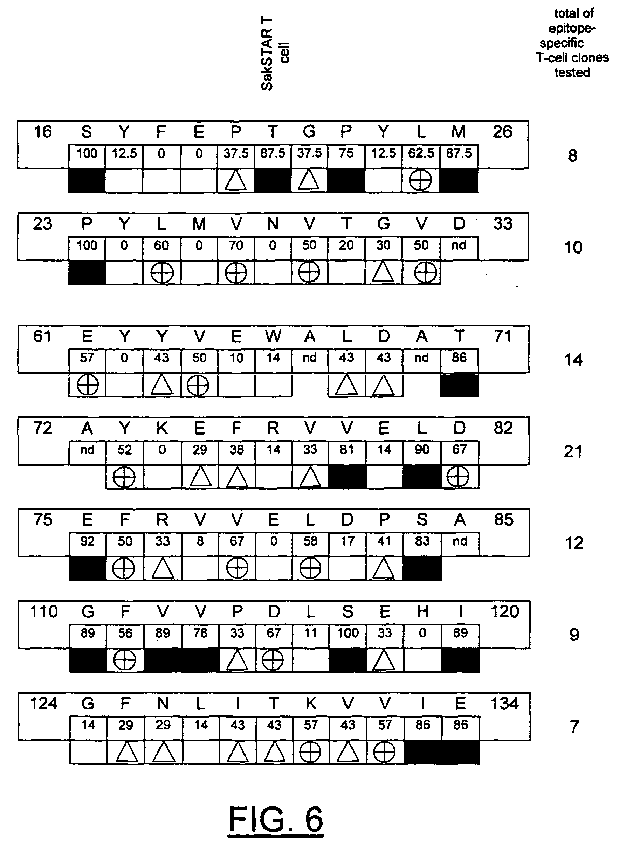

**Figure 6**. Minimal sequence requirements of epitope-specific T-cell clones. The T-cell clones were cultured with APC and according to their specificity with SakSTAR peptides 16-SYFEPTGPYLMVNVTGV-32 (16-26, and 23-33), 56-TKEKIEYYVEWALDATA-72 (61-71), 71-TAYKEFRVVELDPSAKI-87 (72-82, and 75-85), 106-ITEKGFVVPDLSEHIKN-122 (110-120), or 120-IKNPGFNLITKVVIEKK-136 (124-134) combined with single alanine substitution peptides derived from the original SakSTAR peptide. The number of analyzed clones is plotted in the right column. The % of proliferating clones is given for an alanine-substituted peptide at each residue. A dark fill is plotted if more than 75% of the epitope-specific T-cell clones proliferated when the indicated residue was changed into an alanine. A medium gray fill indicates that an alanine change affected less than 25% of the epitope-specific T-cell clones in their proliferative capacity. A light gray box indicates that only 25-50% of the epitope-specific T-cell clones could proliferate, and a white box represents a proliferative response of less than 25% of the epitope-specific T-cell clones. Thus, the whiter the box, the less T-cell clones proliferated to a peptide with an alanine substitution at the indicated position. In summary, a white box indicate that the residue is important for HLA-DR binding or TCR recognition and will be the target for mutagenesis experiments.

### EXAMPLES

### EXAMPLE 1

### Isolation of human staphylokinase-specific T-cell clones

Wild type recombinant staphylokinase (SakSTAR) was expressed and purified as previously described (Collen et al., 1996a; Schlott et al., 1994), followed by an additional size exclusion chromatography step using a superdex 75 column (Pharmacia, Uppsala, Sweden). The purity of SakSTAR was determined to be greater than 98%, as judged by SDS-PAGE analysis.

Normal healthy individuals were HLA-DR typed by the Inno-LiPa method (Buyse et al., 1993) (Innogenetics, Gent, Belgium) and their peripheral blood mononuclear cells (PBMC) isolated according to standard procedures using Ficoll-Hypaque density gradient centrifugation. Ten healthy donors were selected representing >95% of the major HLA-DR haplotypes in the USA and Europe.

For the cloning of SakSTAR-specific T-cell clones, approximately 15x10⁶ PBMC from each donor were stimulated with an optimal concentration of SakSTAR for 7-9 days. The visible T-cell clones were harvested under the microscope, and individually re-stimulated in a 96-well flat-bottom plate with autologous PBMC as antigen presenting cells (APC). These wells were supplemented on day 1 with 20U/ml recombinant human interleukin-2 (IL-2) (Boehringer Mannheim, Mannheim, Germany) and allowed to grow for 8-11 days. Re-stimulation was repeated every 8-11 days under the same conditions. After 3 to 6 passages, autologous PBMC were replaced by autologous Epstein Barr Virus (EBV)-immortalized B-cell lines as antigen presenting cells.

In order to obtain autologous APC which could be maintained for longer periods of time, EBV was isolated from Marmoset B95-8 cells (ATCC#CRL1612) according to standard centrifugation and filtration procedures (Miller and Lipman, 1973).

Concentrated PBMC from the T-cell clone donors were infected for 2 hours with EBV containing supernatants. They were subsequently diluted and cultured at 5.10⁶ PBMC/ml, in RPMI1640 supplemented with 10% FCS, 10 µg/ml gentamycin and 1 µg/ml cyclosporin A. The B-lymphocytes that were transformed with EBV could now be maintained in culture and stored under liquid nitrogen. These procedures resulted in the isolation of over 100 SakSTAR specific T-cell clones (CD3⁺, CD4⁺).

### EXAMPLE 2

### Localization and characterization of immunogenic regions in staphylokinase by functional T-cell assays

To identify immunogenic regions on the SakSTAR molecule, the isolated T-cell clones were tested for their specificity using SakSTAR-derived peptides. In total 25 peptides 17 residues in length, each overlapping 12 residues were synthesized using a fluorenylmethoxycarbonyl-protected amino acid coupling procedure (Hiemstra et al., 1997). Each individual T-cell clone was tested for its specificity by co-culturing T-cells, Mitomycin C-treated autologous EBV-B-cells and antigen or the appropriate peptide for 4 days. Subsequently, the T-cell clones were pulsed for 24 hours with BrdU, harvested and analyzed for their BrdU content. Proliferation after stimulation with a specific peptide was found positive, if the proliferation was at least three times the background, revealing the specificity of that particular T-cell clone.

These assays revealed the presence of 6 distinct immunogenic regions within the SakSTAR molecule. The following peptides (SakSTAR residue number and sequence) were found to be immunogenic:
1-SSSFDKGKYKKGDDASY-17,
16-SYFEPTGPYLMVNVTGV-32,
56-TKEKIEYYVEWALDATA-72,
71-TAYKEFRWELDPSAKI-87,
106-ITEKGFVVPDLSEHIKN-122, and
120-IKNPGFNLITKVVIEKK-136.

### EXAMPLE 3

### Localization and characterization of human T-cell epitopes in staphylokinase by computer modeling of peptides in the HLA-DR binding groove

The total amino acid sequence of SakSTAR was threaded as 11-mer sequences, shifting one residue at the time, through the HLA-DR binding groove of the major haplotypes, and the interaction energy of all these combinations were calculated, using the dead-end elimination theorem (DEE) (De Maeyer et al., 1997; Desmet et al., 1992), which is embedded in the Brugel molecular modeling package (Delhaise et al., 1984).

The energy function (Wodak et al., 1986) used, comprises the usual terms for bond stretching, bond-angle bending, a periodic function for the torsion-angles, a Lennard-Jones potential for the nonbonded atom pairs, a 10-12 potential for hydrogen bonds, and a Coulombic function for charged atoms. The dielectric constant has been set to rᵢⱼ, the distance between the i and j atoms (Warshel and Levitt, 1976). The energy parameters are derived from the CHARMM library (Brooks et al., 1983). All modeling uses the above energy function in the presence of all explicit hydrogen atoms, the carboxylate and imidazole groups were not protonated.

These calculations predicted that SakSTAR contained several T-cell epitopes, which could fit in most HLA-DR haplotypes.

### EXAMPLE 4

### Identification of T-cell epitopes in SakSTAR

Combining the specificity data of the isolated T-cell clones with the predicted T-cell epitopes obtained from computer modeling yielded largely overlapping results. The identified SakSTAR T-cell epitopes are summarized in Table I.

**Table I Overview of the identified T-cell epitopes in staphylokinase; SakSTAR**

| Position in Staphylokinase | Residues in Staphylokinase |
|---|---|
| 6-17 | KGKYKKGDDASY |
| 16-26 | SYFEPTGPYLM |
| 23-33 | PYLMVNVTGVD |
| 43-53 | HYVEFPIKPGT |
| 61-71 | EYYVEWALDAT |
| 65-75 | EWALDATAYKE |
| 72-82 | AYKEFRVVELD |
| 75-85 | EFRVVELDPSA |
| 90-100 | TYYDKNKKKEE |
| 110-120 | GFVVPDLSEHI |
| 124-134 | GFNLITKVVIE |

The numbers represent the residue positions in the mature staphylokinase molecule, the amino acids are represented by a single letter code.

The SakSTAR sequences 43-HYVEFPIKPGT-53 and 90-TYYDKNKKKEE-100 were calculated to fit in most HLA-DR haplotypes, however, no T-cell clones were isolated that proliferated to peptides which included these sequences. The remaining 9 identified epitopes were found to be functional, since epitope-specific T-cell clones were isolated.

### EXAMPLE 5

### Determination of the residue preference at each position in the HLA-DR peptide-binding groove

As described above computer modeling allows the identification of amino acid sequences in a protein with good interaction energy with the binding groove of the HLA-DR molecule, which implies T-cell stimulating capability and therefore immunogenicity.

The same computer technique may be used to model sequences that are less favorable for binding at each position in the peptide-binding groove. The DEE implementation allows the calculation of the interaction energy of the complete enumeration of all sequence combinations for a 9-mer peptide bound in the groove. From these results, the skilled person will be able to extract an interaction preference matrix with the calculation method as described in Example 3. This matrix contains for each amino acid at each position in the peptide the interaction energy with the HLA-DR molecule. This interaction energy is interpreted as the likelihood for an amino acid to be found in a binding peptide.

Therefore, less well binding residues may possibly lower the affinity or even disrupt the binding of the peptide in the HLA molecule. This matrix was calculated for each HLA-DR haplotype. If the DR restriction for an immunogenic region is known, these tables can be consulted to design alternate sequences, which bind less well in the groove, but do not interfere with the 3-D folding of the protein. Since the different DR matrices share a common pattern, a generalized matrix can be assembled. In case the DR restriction is not known, this generalized matrix can be consulted.

### EXAMPLE 6

### Identification of critical residues in an immunogenic region by screening with a T-cell clone library

Interestingly, the peptide with sequence 71-TAYKEFRWELDPSAKI-87 of SakSTAR stimulated T-cell clones isolated from 9 of 10 donors, suggesting a promiscuous character. In order to define the minimal epitope for each individual T-cell clone, SakSTAR region(71-87)-peptide derivatives, each one with a different single alanine substitution were used in T-cell proliferation assays. A representative panel is shown in Fig. 2.

Alanine substitution at positions F76, R77, V78, or E80, abolished proliferation of the majority of the region(71-87)-specific T-cell clones. Side chain removal by mutation into an alanine residue of K74 or D82 affected the proliferative capacity of approximately half of the region(71-87)-specific T-cell clones, whereas a minority of them was found sensitive for an alanine substitution of residues Y73, E75, V79, L81 or P83.

### EXAMPLE 7

### Analysis of an immunogenic region by computer analysis

T-lymphocyte proliferation requires both appropriate peptide presentation and T-cell receptor recognition. Consequently, T-cell assays alone cannot discriminate between HLA-DR-binding and receptor-facing residues within a peptide. To unravel the molecular basis of this ambiguity, a computer modeling approach was used starting from available HLA-DR1 X-ray coordinates (Brown et al., 1993). The extended immunogenic SakSTAR sequence 67-ALDATAYKEFRVVELDPSAKIEV-89 was threaded as 11-mer peptides, each shifted by a single residue, through the HLA-DR1 peptide-binding groove. The main-chain atoms of both HLA-DR and the peptide were kept fixed, while all side chains were placed in the global minimum energy conformation using the dead-end elimination theorem (Desmet et al., 1992). Modeling in the context of the other haplotypes, yielded essentially the same results. The interaction energy for each peptide with the groove was calculated, and is represented in Fig.3a.

Two strong binding sequences AYKEFRVVELD (S1; SakSTAR residues 72-82) and EFRVVELDPSA (S2; SakSTAR residues 75-85) were identified (Fig.3a; black bars), placing the second residue of both sequences in HLA-DR pocket 1. A typical model of the S2 peptide within the binding groove is shown in Fig.3b.

The extent of both solvent exposure and burying of each residue of the S1 and S2 peptides in the HLA-DR groove is shown in Fig.4c and 4f respectively. The large binding pocket 1 harbors an aromatic residue Y73 (in S1) or F76 (in S2) making buried aromatic contacts. Pocket 4 is filled either with an aromatic F76 (in S1) or a hydrophobic residue V79 (in S2), while the hydrophobic residues V78 (in S1) and L81 (in S2) are pointing into pocket 6. Although both residues V79 (in S1) and D82 (in S2) bury their side chain in pocket 7, the contribution to the interaction energy of S2 is more of an electrostatic nature via residue 71 of the HLA-DR β chain, which in most haplotypes is an arginine. The corresponding contribution of L81 (in S1) to the contact area of pocket 9 is less obvious for the S2 peptide were a serine is found. Furthermore, these observations suggest that while the buried peptide residues predominantly contribute to HLA-DR anchoring, the exposed residues mediate both T-cell receptor recognition and the binding to HLA-DR.

### EXAMPLE 8

### Identification of the minimal epitopes recognized by the region(71-87) T-cell clones

To confirm that both predicted sequences were functional T-cell epitopes, the minimal S1 and S2 11-mer sequences were synthesized and used in proliferation assays with the region(71-87)-specific T-cell clones.

Fig. 2 shows that clones Tc-1 to Tc-6 proliferated after being challenged with the S1 peptide, but not with the S2 peptide. Tc-7 to Tc-10 recognize the S2 peptide but not the S1 peptide. These results indicate that both S1 and S2 are functional T-cell epitopes.

### EXAMPLE 9

### In silico alanine scan of immunogenic regions

The contribution of each residue to the HLA-DR binding was calculated for both sequences by an in silico alanine scan. The scan of the S1 residues revealed a correlation between the loss of interaction energy and the extent of burying in HLA-DR pockets 1, 4 and 6 (Fig. 4a,c). The residues E75 or E80, respectively at positions 3 and 8 in the S1 sequence are not deeply buried in the groove, however an alanine substitution was calculated to result in a substantial loss in interaction energy. The largest contact loss between the S1 peptide and the groove was obtained with a side-chain removal of the residues occupying the pockets 1, 4 or 9 (Fig. 4b). For an alanine substitution of the residues F76, R77 and L81, at positions 1, 2 and 6 respectively in the S2 mode (Fig. 4d-f), an important loss of interaction energy and a proportional loss of contact area was observed. Alanine substitution in pocket 7 of the S2 sequence (D82) revealed no major loss in contact area, however a substantial energy loss was found, which may probably be due to the removal of the ionic interaction with residue R71 of the HLA-DR-β chain (see also Fig.3b).

### EXAMPLE 10

### Mutagenesis in the immunogenic SakSTAR region 71-87

Altogether, these observations show that mutations at a combination of key positions lead to a reduced immunogenicity of this sequence. Changes into alanine of the important anchoring residues of both epitopes at amino acid positions Y73, F76, V78, and L81 abolished the plasminogen activating potency.

Therefore, other residues that were important for T-cell proliferation (Fig. 2) and that contributed to HLA-DR binding were targeted (Fig. 4). Residues K74 and E80 were chosen for their dual contribution in DR-anchoring and facing the TCR in the S1 binding mode. This dual contribution was also found for residue R77 in the S2 binding mode. Furthermore, residues R77 and E80 were found to be solvent exposed at an important TCR recognition position (pocket 5) in the S1 and S2 peptide, respectively. Finally, D82 was targeted for its anchoring contribution in the S2 binding mode.

The SakSTAR-mutants were engineered by the spliced overlap extension polymerase chain reaction (SOE-PCR) (Horton et al., 1989) using as template pMEX.SakSTAR-myc, encoding the wild-type SakSTAR sequence with a carboxy-terminal additional triple alanine followed by a myc sequence (Evan et al., 1985). For each mutant, the amplified product was purified and ligated into the pMEX vector (Schlott et al., 1994). All constructions were confirmed by sequencing of the entire SakSTAR-myc coding region.

The SakSTAR-variants were expressed and purified from transformed E. coli TG1, using experimental conditions as described (Collen et al., 1996a), followed by size exclusion chromatography using a superdex 75 column (Pharmacia, Uppsala, Sweden). The purity of the SakSTAR variants was confirmed by SDS-PAGE to be at least 98%. The following SakSTAR mutants were constructed, SakSTAR(R77A, E80A), SakSTAR(R77A, E80A, D82A) and SakSTAR(K74Q, R77S, E80S, D82S). The specific activity of all these mutants was between 50% and 100% of that of wild-type SakSTAR. Variants of wild-type staphylokinase are identified herein by the substituted amino acids in single letter symbols followed by their position number in the mature SakSTAR sequence (136 amino acids) and by the substituting amino acids in single letter symbols.

### EXAMPLE 11

### Immunogenicity analysis of the redesigned 71-87 region of SakSTAR

The S1 and S2 binding mode of the above SakSTAR mutants were remodeled in the HLA-DR binding groove. A loss of at least 15 kcal/mol in interaction energy and 40 Å² in contact area was found for the S1 peptide of any of these variants. For the S2 stretch, a loss in interaction energy was found to be at least 10 kcal/mol, and over 100 Ä² in the contact area (data not shown), indicating a reduced binding of the mutant peptide sequences with HLA-DR.

In addition, the area 69-89 of the SakSTAR variants were threaded through the binding groove similarly as shown in Fig. 4a to analyze the possibility of newly introduced epitopes. Importantly, no new binding motifs in any of the SakSTAR-variants were revealed. All region(71-87)-specific T-cell clones were tested for proliferation after challenge with the above described SakSTAR-mutants, and none of them showed proliferation. This was not due to presentation problems, because T-cell clones recognizing a different SakSTAR epitope were perfectly capable to proliferate after stimulation with either region(71-87) SakSTAR variant.

### EXAMPLE 12

### Cellular immune response to the newly designed molecules

The human T-cells used in the functional assays were obtained by selection and cloning procedures. Consequently, they may represent only a subset of region(71-87)-specific T-cells. To investigate whether a redesigned region actually results in a reduced immunogenic protein, a cellular immune response to a redesigned protein should be compared to that of its wild type counterpart.

However, the vast majority of humans does also recognize additional immunogenic SakSTAR regions (untouched in these SakSTAR-variants), which would invalidate such an experiment. Therefore, 25 other healthy donors were screened for SakSTAR immunoreactivity. PBMC were thawed, washed four times in 1% human serum albumin, and cultured at 1.8x10⁶ cells/ml in RPMI 1640 supplemented with 5% human serum (Biowitthaker, Walkersville, Maryland) and gentamycin with or without SakSTAR-myc for 10 days in a humidified CO₂ incubator. The non-adherent cells were harvested and restimulated with no antigen, SakSTAR-myc or different peptides. For antigen presenting cells, irradiated autologous PBMC were T-cell depleted, using CD3-M450 Dynabeads (Dynal, Compiégne, France) according manufacturer procedures (FACS analysis indicated >95% non-T-cells). The plates were incubated at 37°C under humidified atmosphere containing 5% CO₂ for 5 days, and pulsed with BrdU for the last 22 to 24 hours.

Subsequently, the cells were harvested and analyzed for their BrdU content (BrdU ELISA, Boehringer Mannheim, Mannheim, Germany). A stimulation index was calculated, by dividing the OD obtained from the PBMC in response to an antigen/peptide by that of the spontaneous proliferation (no antigen) control. A value exceeding 3 was considered positive, indicating the proliferation of specific T-cells to that particular antigen/peptide.

A cellular immune response to the SakSTAR peptide(71-87) was observed in 80% of the 20 identified SakSTAR positive donors, confirming its immunodominant character.

PBMC from the 10 donors with the highest region(71-87)-specific cellular response were primed with SakSTAR-myc and subsequently challenged with different SakSTAR or region-(71-87)-mutant peptides as indicated in Fig. 5.

Although the overall cellular response to the single alanine mutated peptides was significantly reduced as compared to the wild-type peptide (P≤0.05), these peptides induced a cellular response in several of the tested donors. The peptides with an alanine at positions R77 or E80 hampered the lymphocyte proliferation of the majority of the tested donors. The relative importance of these residues was also found in the proliferation assays with the region-(71-87)-specific T-cell clones.

However, if SakSTAR-myc primed lymphocytes are restimulated with the double (R77A, E80A), triple (R77A, E80A, D82A), quadruple (K74Q, R77S, E80S, D82S) or quintuple (K74A, E75A, R77A, E80A, D82A) mutated peptides, none of the tested donors showed a positive cellular immune response (P≤0.001). This implicates that region(71-87)-specific circulating T-lymphocytes in humans (which may have been generated by earlier infections with lysogenic Staphylococcus strains) are no longer capable of recognizing the mutated region 71-87 of any of the SakSTAR variants. Consequently, their proliferation and help to support B-lymphocytes to generate antibodies is impaired.

### EXAMPLE 13

### Identification of critical residues in the other identified T-cell epitopes in the SakSTAR molecule

T-cell clones recognizing several of the other SakSTAR epitopes (see Table I) were analyzed in a similar manner as the region(71-87)-specific T-cell clones described in the preceding examples. The minimal sequence requirements for most SakSTAR-specific T-cell clones was determined in T-cell proliferation assays, using peptides each containing a different single alanine substitution. The results are summarized in Figure 6.

The T-cell clones that proliferated after a challenge with the peptide 16-SYFEPTGPYLMVNVTGV-32 could be divided into two groups, 8 T-cell clones recognized the epitope 16-26 and 10 clones did recognize the SakSTAR sequence 23-33. An alanine substitution at F18, or E19 abrogated proliferation of all isolated region(16-26)-specific T-cell clones, whereas most T-cell clones could no longer proliferate if Y17, or Y24 was mutated into an alanine. Side-chain removal of P20 or G23 affected the T-cell proliferation of more than half of this group of T-cell clones. All isolated region(23-33)-specific T-cell clones were found sensitive for an alanine substitution at positions Y24, M26, or N28, whereas an alanine substitution of T30 or G31 abrogated proliferation of the majority of the isolated region(16-26)-specific T-cell clones.

Based on these data combined with the calculated residue preference matrices several SakSTAR-mutants were constructed: SakSTAR(P20Y, Y24A), SakSTAR(P20Y, Y24S), SakSTAR(E19D), SakSTAR(Y17L), SakSTAR(F18L), SakSTAR(F18E), SakSTAR(G22S), SakSTAR(G22S, P23G), SakSTAR(N28S), and SakSTAR(V29L, L127V). All these SakSTAR-mutants had a reduced immunogenic profile for the region(16-32) specific T-cell clones.

Analysis of 14 T-cell clones specific for the SakSTAR peptide 56-TKEKIEYYVEWALDATA-72 was performed. An alanine substitution at residues Y62, E65 or W66 resulted in the loss of proliferation of the vast majority of these region-specific T-cell clones. Approximately half of these T-cell clones were found sensitive for side chain removal of residues E61, Y63, V64, L68 or D69. Based on these data combined with the calculated residue preference matrices several SakSTAR-mutants were constructed, however all had lost their thrombolytic activity. The 56-72 immunogenic region of SakSTAR is part of the activation epitope as defined by Jespers et al. Mutation of one of the activation epitope residues into most other residue types creates a biological inactive molecule (Jespers et al (1999)).

Nine T-cell clones specific for the SakSTAR peptide 106-ITEKGFVVPDLSEHIKN-122 were also analyzed. Alanine substitution at positions P114, L116, E118, or H119 abolished proliferation of the majority of the region(106-122)-specific T-cell clones. Side chain removal of F111 or D115 affected the proliferative capacity of approximately half of these T-cell clones. Based on these data combined with the calculated residue preference matrices several SakSTAR-mutants were constructed: SakSTAR(H119A), SakSTAR(H119S), SakSTAR(V89L, L116Y), SakSTAR(V89L, L116T), SakSTAR(V112T), SakSTAR(V112S), SakSTAR(D115N), and SakSTAR(E118S). All these SakSTAR-mutants had a significantly reduced immunogenic profile for the region(106-122) specific T-cell clones. Although an alanine change at V112 had an effect of only 11% of the region-specific T-cell clones, interestingly the biologically active mutants SakSTAR(V112T) and SakSTAR(V112S) could not induce proliferation in any of the region(106-122)-specific T-cell clones. This was not due to the construction (as confirmed by sequencing) or purity of the isolated SakSTAR mutant, because T-cell proliferation of other SakSTAR-specific T-cell clones could be induced using these mutants comparable to that with wild-type SakSTAR.

The following combinatorial mutants have been constructed and isolated; SakSTAR(V112T, H119A), SakSTAR(V112T, H119S), SakSTAR(V112T, E118S, H119S), and SakSTAR(V112T, H119S, K130Y). These thrombolytically active mutants lost their capacity to induce the region(106-122)-specific T-cell clone proliferation, indicating that the immunogenicity of SakSTAR region 106-122 was eliminated.

Finally, 7 T-cell clones recognizing the SakSTAR peptide 120-IKNPGFNLITKVVIEKK-136 were analyzed for their minimal sequence requirements. Alanine substitution at positions G124, F125, N126, or L127 abolished proliferation of the majority of the region(120-136)-specific T-cell clones. Side chain removal of I128, T129, K130, V131, or V132 affected the proliferative capacity of approximately half of these T-cell clones. Based on these data combined with the calculated residue preference matrices, two biologically active SakSTAR-mutants were constructed. The mutant SakSTAR(R77E, E134R) has been tested in proliferation assays using several region(120-136)-specific T-cell clones and some region(71-89)-specific T-cell clones. It was found that this mutant could support the proliferation of some, but not all T-cell clones from both specificities, indicating that two SakSTAR regions had a reduced immunogenic profile. Although the mutation of residue K130 into alanine in the SakSTAR120-136 peptide did only have an effect on approximately half of the isolated region(120-136)-specific T-cell clones, this residue was targeted. Computer modeling supplemented with the residue preference matrix revealed that if an Y replaces residue K130 within this epitope (GFLIT**Y**VVIE), the interaction energy between this mutant-epitope and the HLA-DR binding groove is so high, that binding within the groove is predicted to be impossible. Therefore, mutant SakSTAR(K130Y) was constructed, expressed and purified, and was found to have a thrombolytic activity similar to that of wild-type SakSTAR. The region(120-136)-specific T-cell clones were tested with this SakSTAR-mutant, and none of them could proliferate, suggesting the epitope was no longer functional. Therefore, mutation of residue K130 of SakSTAR into Y is the ultimate mutation, because the presentation of this redesigned region is completely lost.

Altogether, these analyses demonstrate that the immunogenicity of a heterologous protein can be reduced by the removal of functional T-cell epitopes. As a consequence, the elimination of a combination of immunogenic regions will further reduce the T-cell reactivity, and therefore the overall immunogenicity of a combined mutant protein. Furthermore, the rational approach combining functional assays with mathematical models may lead to the precise removal of HLA-DR anchoring residues, affecting the presentation of T-cell epitopes and the initiation of T-cell reactivity.

### REFERENCES

Behnke, D., and Gerlach, D. (1987). Cloning and expression in Escherichia coli, Bacillus subtilis, and Streptococcus sanguis of a gene for staphylokinase--a bacterial plasminogen activator. Mol Gen Genet 210, 528-534.
Brooks, B. R., Bruccoleri, R., Olafson, D., States, D. J., Swaminathan, S., and Karplus, M. (1983). CHARMM: A Program for macromolecular energy, minimization, and dynamics calculations. J. Comput. Chem 4, 187-217.
Brown, J. H., Jardetzky, T. S., Gorga, J. C., Stern, L. J., Urban, R. G., Strominger, J. L., and Wiley, D. C. (1993). Three-dimensional structure of the human class II histocompatibility antigen HLA-DR1. Nature 364, 33-39.
Buyse, I., Decorte, R., Baens, M., Cuppens, H., Semana, G., Emonds, M. P., Marynen, P., and Cassiman, J. J. (1993). Rapid DNA typing of class II HLA antigens using the polymerase chain reaction and reverse dot blot hybridization. Tissue Antigens 41, 1-14.
Collen, D. (1998). Staphylokinase: a potent, uniquely fibrin-selective thrombolytic agent. Nat Med 4, 279-284.
Collen, D., Bernaerts, R., Declerck, P., De Cock, F., Demarsin, E., Jenne, S., Laroche, Y., Lijnen, H. R., Silence, K., and Verstreken, M. (1996a). Recombinant staphylokinase variants with altered immunoreactivity. I: Construction and characterization. Circulation 94, 197-206.
Collen, D., Moreau, H., Stockx, L., and Vanderschueren, S. (1996b). Recombinant staphylokinase variants with altered immunoreactivity. II: Thrombolytic properties and antibody induction. Circulation 94, 207-216.
Collen, D., Zhao, Z. A., Holvoet, P., and Marynen, P. (1992). Primary structure and gene structure of staphylokinase. Fibrinolysis 6, 226-231.
De Maeyer, M., Desmet, J., and Lasters, I. (1997). All in one: a highly detailed rotamer library improves both accuracy and speed in the modelling of side chains by dead-end elimination. Fold Des 2, 53-66.
Delhaise, P., Bardiaux, M., and Wodak, S. (1984). Interactive computer animation of macromolecules. J Mol Graphics 2, 103-106.
Desmet, J., De Maeyer, M., Hazes, B., and Lasters, I. (1992). The dead-end elimination theorem and its use in protein side-chain positioning. Nature 356, 539-542.
Evan, G. I., Lewis, G. K., Ramsay, G., and Bishop, J. M. (1985). Isolation of monoclonal antibodies specific for human c-myc proto-oncogene product. Mol Cell Biol 5, 3610-3616.
Hiemstra, H. S., Duinkerken, G., Benckhuijsen, W. E., Amons, R., de Vries, R. R., Roep, B. O., and Drijfhout, J. W. (1997). The identification of CD4+ T-cell epitopes with dedicated synthetic peptide libraries. Proc Natl Acad Sci U S A 94, 10313-10318.
Horton, R. M., Hunt, H. D., Ho, S. N., Pullen, J. K., and Pease, L. R. (1989). Engineering hybrid genes without the use of restriction enzymes: gene splicing by overlap extension. Gene 77, 61-68.
Jespers, L., Vanwetswinkel, S., Lijnen, R., Van Herzeele, N., Van Hoef, B., Demarsin, E., Collen, D., and De Maeyer, M. (1999). Structural and functional basis of plasminogen activation by staphylokinase. Thrombosis Hemostasis 81, 479-485.
Koradi, R., Billeter, M., and Wuthrich, K. (1996). MOLMOL: a program for display and analysis of macromolecular structures. J Mol Graphics 14, 51-55.
Miller, G., and Lipman, M. (1973). Release of infectious Epstein-Barr virus by transformed marmoset leukocytes. Proc Natl Acad Sci U S A 70, 190-194.
Sako, T., and Tsuchida, N. (1983). Nucleotide sequence of the staphylokinase gene from Staphylococcus aureus. Nucleic Acids Res 11, 7679-7693.
Schlott, B., Hartmann, M., Guhrs, K. H., Birch-Hirschfeid, E., Pohl, H. D., Vanderschueren, S., Van de Werf, F., Michoel, A., Collen, D., and Behnke, D. (1994). High yield production and purification of recombinant staphylokinase for thrombolytic therapy. Biotechnology (N Y) 12, 185-189.
Warshel, A., and Levitt, M. (1976). Theoretical studies of enzymic reactions: dielectric, electrostatic and steric stabilization of the carbonium ion in the reaction of lysozyme. J Mol Biol 103, 227-249.
Wodak, S. J., De Coen, J. L., Edelstein, S. J., Demarne, H., and Beuzard, Y. (1986). Modification of human hemoglobin by glutathione. III. Perturbations of hemoglobin conformation analyzed by computer modeling. J Biol Chem 261, 14717-14724.

## Claims

1. Method for reducing the immunogenicity of staphylokinase, which method comprises the steps of:
a) designing a series of overlapping test peptides each having an amino acid sequence that corresponds with part of the amino acid sequence of staphylokinase;
b) identifying which of the test peptides of the series comprises one or more T-cell epitope(s);
c) modifying the amino acid sequence of one or more of the test peptides comprising one or more T-cell epitope(s);
d) repeating step b) and optionally step c) with the modified test peptides until one or more of the T-cell epitopes originally comprised therein are significantly reduced or eliminated;
e) modifying the amino acid sequence of staphylokinase according to the T-cell eliminating modifications made in the amino acid sequence of the test peptides to produce a modified staphylokinase,
**characterized in that**
the identification of test peptides comprising a T-cell epitope is performed by a combination of the following tests:
1) a functional T-cell assay in which proliferation of one or more T-cell clones after stimulation of the T-cell clone(s) with one of the test peptides means that the said test peptide comprises a potential T-cell epitope; and/or
2) a functional T-cell assay in which proliferation of one or more T-lymphocytes as present in the circulation of humans (PBMC) after stimulation of the PBMC with one of the test peptides means that the said test peptide comprises a T-cell epitope; and
3) determining the interaction energy of the said test peptide with the binding groove of one or more HLA-DR haplotypes by means of computer modeling, in which the test peptide is identified as comprising a potential T-cell epitope when according to its interaction energy it fits into the HLA-DR binding groove,
wherein a test peptide is considered to comprise a T-cell epitope if it is identified in test 1) and/or 2) and 3).

2. Method as claimed in claim 1, further comprising one or more repetitions of steps b) to e) with the modified staphylokinase instead of the test peptides.

3. Method as claimed in claims 1 or 2, which method comprises the steps of:
a) designing a series of overlapping test peptides each having an amino acid sequence that corresponds with part of the amino acid sequence of staphylokinase of which the immunogenicity is to be reduced;
b) identifying which of the test peptides of the series comprises one or more T-cell epitope(s) by means of test 1) and/or 2) and 3) as defined in claim 1;
c) modifying the amino acid sequence of one or more of the test peptides comprising one or more T-cell epitope(s);
d) testing the modified peptides for their potential to activate T-cells and induce proliferation of either T-cell clones or T-lymphocytes as present in the circulation of humans (PBMC);
e) modifying the amino acid sequence of the staphylokinase of which the immunogenicity is to be reduced according to the T-cell eliminating modifications made in the amino acid sequence of the test peptides;
f) construction and purification of the modified staphylokinase of which the immunogenicity is to be reduced in a suitable host, and testing its biological activity;
g) repeating step c-f) with the modified test peptides or staphylokinase until one or more of the T-cell epitopes originally comprised therein are significantly reduced or eliminated and contemplating the biological activity.

4. Method for reducing the immunogenicity of staphylokinase, which method comprises the steps of:
a) designing a series of overlapping test peptides each having an amino acid sequence that corresponds with part of the amino acid sequence of the staphylokinase of which the immunogenicity is to be reduced;
b) identifying which of the test peptides of the series comprises one or more T-cell epitope(s) by means of test 1) and/or 2) and 3) as defined in claim 1;
c) modifying the amino acid sequence of one or more of the test peptides comprising one or more T-cell epitope(s);
d) testing the modified peptides for their potential to activate T-cells and induce proliferation of either T-cell clones and/or T-lymphocytes as present in the circulation of humans (PBMC);
e) modifying the amino acid sequence of staphylokinase of which the immunogenicity is to be reduced according to the T-cell eliminating modifications made in the amino acid sequence of the test peptides;
f) construction and purification of the modified staphylokinase of which the immunogenicity is to be reduced in a suitable host, and testing its biological activity;
g) repeating step c-f) with the modified test peptides or staphylokinase until one or more of the T-cell epitopes originally comprised therein are significantly reduced or eliminated and contemplating the biological activity.

5. Method as claimed in claims 1-4, wherein the T-cell epitopes are eliminated by minimal residue/region mutagenesis.

6. Method as claimed in claims 1-5, wherein the T-cell epitopes are eliminated by deletion or insertion of residues in the identified T-cell epitopes.

7. Method as claimed in claims 1-6, wherein the T-cell epitopes are eliminated by substitution with human sequences, or human homologous sequences.

8. Method as claimed in claims 1-7, wherein the T-cell epitopes are eliminated by enhancing the sensitivity of the identified T-cell epitopes for proteolytic endosomal enzymes.

9. Method as claimed in claims 1-8, further comprising the identification and removal of HLA-DR anchoring residues of staphylokinase to reduce the immunogenicity thereof.

10. Method as claimed in claims 1-9, wherein the biological activity of staphylokinase with reduced immunogenicity is tested after modification of the amino acid sequence of staphylokinase.

11. Method for producing a staphylokinase having a reduced immunogenicity in comparison to the wild type staphylokinase, comprising the following steps:
a) providing a DNA sequence encoding at least the amino acid sequence of staphylokinase that provides for the biological activity thereof;
b) identifying one or more of the T-cell epitopes comprised in the amino acid sequence of the wild type staphylokinase by:
1) designing a series of overlapping test peptides each having an amino acid sequence that corresponds with part of the amino acid sequence of staphylokinase;
2) identifying which of the test peptides of the series comprises one or more T-cell epitope(s) by means of test 1) and/or 2) and 3) as defined in claim 1;
3) modifying the amino acid sequence of one or more of the test peptides comprising one or more T-cell epitope(s);
4) repeating step 2) and optionally step 3) with the modified test peptides until one or more of the T-cell epitopes originally comprised therein are significantly reduced or eliminated;
c) modifying the DNA sequence to introduce the modifications to the T-cell epitope(s) in the amino acid sequence encoded thereby;
d) introducing the DNA sequence into a host organism;
e) culturing the host organism under conditions allowing expression of the modified staphylokinase; and
f) isolating the staphylokinase having a reduced immunogenicity from the host organism.

12. Method for producing a staphylokinase having a reduced immunogenicity in comparison to the wild type staphylokinase, comprising the following steps:
a) providing a DNA sequence encoding at least the amino acid sequence of staphylokinase that provides for the biological activity thereof;
b) identifying and modifying one or more of the T-cell epitopes comprised in the amino acid sequence of the wild type staphylokinase by:
1) identifying which of the test peptides of the series comprises one or more T-cell epitope(s) by means of test 1) and/or 2) and 3) as defined in claim 1;
2) modifying the amino acid sequence of one or more of the test peptides comprising one or more T-cell epitope(s);
3) testing the modified peptides for their potential to activate T-cells and induce proliferation of either T-cell clones or T-lymphocytes as present in the circulation of humans (PBMC);
4) modifying the amino acid sequence of the staphylokinase of which the immunogenicity is to be reduced according to the T-cell eliminating modifications made in the amino acid sequence of the test peptides;
5) construction and purification of the modified staphylokinase of which the immunogenicity is to be reduced in a suitable host, and testing its biological activity;
6) repeating step 2-5) with the modified staphylokinase until one or more of the T-cell epitopes originally comprised therein are significantly reduced or eliminated and contemplating the biological activity;
c) modifying the DNA sequence to introduce the modifications to the T-cell epitope(s) in the amino acid sequence encoded thereby;
d) introducing the DNA sequence into a host organism;
e) culturing the host organism under conditions allowing expression of the modified staphylokinase; and
f) isolating the staphylokinase having a reduced immunogenicity from the host organism.

13. Staphylokinase variants in which one or more of the following immunogenic regions are modified such that the T cell epitopes contained therein are eliminated:
1-SSSFDKGKYKKGDDASY-17,
16-SYFEPTGPYLMVNVTGV-32,
56-TKEKIEYYVEWALDATA-72,
71-TAYKEFRVVELDPSAKI-87,
106-ITEKGFVVPDLSEHIKN-122, and
120-IKNPGFNLITKVVIEKK-136
excluding the variant having the combination of the following mutations: K74A, E75A, R77A, E80A, D82A.

14. Staphylokinase variants as claimed in claim 13 with reduced T-cell reactivity as compared to wild type staphylokinase, but retaining its biological activity, which variants have the amino acid sequence depicted in Figure 1 with one or more of the following amino acid substitutions in one or more of the following immunogenic region:
a) in the immunogenic region comprising SakSTAR residues 16-32 the substitutions Y17L; F18L; F18E; E19D; P20Y, Y24A; P20Y, Y24S; G22S; G22P, P23G; or N28S;
b) in immunogenic region comprising SakSTAR residues 71-82 the substitutions F76W, V79Y, D82A; R77A, E80A; R77S, E80S; R77A, E80A, D82A; K74Q, R77S, E80S; K74Q, R77S, E80S, D82G; K74Q, R77S, E80S, D82A; K74Q, R77S, E80A, D82A; K74Q, R77A, E80S, D82A; K74Q, R77S, E80S, D82S; or V79Y, D82A;
c) in immunogenic region comprising SakSTAR residues 106-122 the substitutions V112T; V112S; D115N; E118S; H119A; H119S; V89L, L116Y; V89L, L116T; V112T, H119S; V112T, H119A; or V112T, E118S, H119S;
d) in immunogenic region comprising SakSTAR residues 120-136, the substitution K130Y.

15. Staphylokinase variants as claimed in claim 14, having a combination of one or more of the mutations listed in claim 14.

16. Staphylokinase variants as claimed in claim 15, having one of the following combination of mutations
V112T, H119S, K130Y; R77E, E134R; V29L, L127V; K74Q, R77E, E80S, D82S, E134R; R77S, E80S, V112T, H119S; R77S, E80S, V112T, H119S, K130Y; R77A, E80A, V112T, H119S; R77A, E80A, V112T, H119S, K130Y; K74Q, R77S, E80S, V112T, H119S; K74Q, R77S, E80S, V112T, H119S, K130Y; K74Q, R77S, E80S, D82S, V112T, H119S; or K74Q, R77S, E80S, D82S, V112T, H119S, K130Y.

17. Staphylokinase variant selected from the group consisting of SakSTAR(P20Y, Y24A), SakSTAR(P20Y, Y24S), SakSTAR(E19D), SakSTAR(Y17L), SakSTAR(F18L), SakSTAR(F18E), SakSTAR(G22S), SakSTAR(G22S, P23G), SakSTAR(N28S), SakSTAR(V29L, L127V), SakSTAR(R77E, E134R), SakSTAR(K74Q, R77E, E80S, D82S, E134R), sakSTAR(K74Q, R77E, E80S, D82S,V112T, H119S, E134R), sakSTAR(K74Q, R77E, E80S, D82S,V112T, H119S, E134R, K130Y), SakSTAR(R77S, E80S, V112T, H119S), SakSTAR(R77S, E80S, V112T, H119S, K130Y), SakSTAR(R77A, E80A, V112T, H119S), SakSTAR(R77A, E80A, V112T, H119S, K130Y), SakSTAR(K74Q, R77S, E80S, V112T, H119S), SakSTAR(K74Q, R77S, E80S, V112T, H119S, K130Y), SakSTAR(K74Q, R77S, E80S, D82S, V112T, H119S), SakSTAR(K74Q, R77S, E80S, D82S, V112T, H119S, K130Y), SakSTAR(V112T, H119A), SakSTAR(V112T, H1193), SakSTAR(V112T, E118S, H119S), SakSTAR(V112T, H119S, K130Y), SakSTAR(V112T), SakSTAR(V112S), SakSTAR(H119A), SakSTAR(H119S), SakSTAR(V89L, L116Y), SakSTAR(V89L, L116T), SakSTAR(V112T), SakSTAR(V112S), SakSTAR(D115N), SakSTAR(E118S), SakSTAR(K130Y).

18. Staphylokinase variant as claimed in claims 13-17 for use in treatment, diagnosis or prophylaxis.

19. Use of a staphylokinase variant as claimed in claims 13-17 for the preparation of a pharmaceutical composition for treatment, diagnosis or prophylaxis of a human subject.

20. Pharmaceutical composition comprising a staphylokinase variant as claimed in claims 13-17 together with a pharmaceutically acceptable carrier, diluents or excipient.

## Patentansprüche

1. Verfahren zum Verringern der Immunogenität von Staphylokinase, wobei das Verfahren die Schritte umfasst:
a) Entwurf einer Reihe von überlappenden Testpeptiden von denen jedes eine Aminosäuresequenz besitzt, die einem Teil der Aminosäuresequenz von Staphylokinase entspricht;
b) Identifizieren welches der Testpeptide der Reihe ein oder mehrere T-Zell-Epitope umfasst;
c) Modifizieren der Aminosäuresequenz eines oder mehrerer Testpeptide, die ein oder mehrere T-Zell-Epitope umfassen;
d) Wiederholen des Schritts b) und wahlweise Schritts c) mit den modifizierten Testpeptiden, bis eines oder mehrere der T-Zell-Epitope, die ursprünglich darin enthalten sind, deutlich verringert oder beseitigt ist;
e) Modifizieren der Aminosäuresequenz von Staphylokinase gemäß den T-Zell beseitigenden Modifikationen, die in der Aminosäuresequenz der Testpeptide durchgeführt wurden, um eine modifizierte Staphylokinase herzustellen,
**dadurch gekennzeichnet, dass**
die Bestimmung der Testpeptide, die ein T-Zell-Epitop enthalten, durch eine Kombination der folgenden Tests durchgeführt wird:
1) funktioneller T-Zellassay, in dem die Proliferation von einem oder mehreren T-Zellklonen nach Stimulation der T-Zellklone mit einem der Testpeptide bedeutet, dass das Testpeptid ein potentielles T-Zell-Epitop umfasst; und/oder
2) funktioneller T-Zellassay, in dem die Proliferation von einem oder mehreren T-Lymphozyten, wie sie in dem Kreislauf von Menschen (PBMC) nach der Stimulation des PBMC mit einem der Testpeptide vorhanden sind, bedeutet, dass das Testpeptid ein T-Zell-Epitop enthält; und
3) Bestimmen der Interaktionsenergie des Testpeptids mit der Bindungsfurche eines oder mehrerer HLA-DR Haplotypen durch Computermodellierung, in denen das Testpeptid als ein potentielles T-Zell-Epitop enthaltendes Peptid identifiziert wird, wenn es gemäß seiner Interaktionsenergie in die HLA-DR Bindungsfurche passt, wobei angenommen wird, dass ein Testpeptid ein T-Zell-Epitop umfasst, wenn es in Test 1) und/oder 2) und 3) identifiziert wird.

2. Verfahren wie in Anspruch 1 beansprucht, ferner umfassend ein oder mehrere Wiederholungen der Schritte b) bis e) mit der modifizierten Staphylokinase anstelle der Testpeptide.

3. Verfahren wie in den Ansprüchen 1 oder 2 beansprucht, wobei das Verfahren die Schritte umfasst:
a) Entwurf einer Reihe von überlappenden Testpeptiden, von denen jedes eine Aminosäuresequenz besitzt, die einem Teil der Aminosäuresequenz der Staphylokinase deren Immunogenität reduziert werden soll, entspricht;
b) Identifizieren welches der Testpeptide der Reihe ein oder mehrere T-Zell Epitope umfasst durch Test 1) und/oder 2) und 3) wie in Anspruch 1 definiert;
c) Modifizieren der Aminosäuresequenz eines oder mehrerer Testpeptide, die ein oder mehr T-Zell-Epitope enthalten;
d) Testen der modifizierten Peptide auf ihr Potential T-Zellen zu aktivieren und die Proliferation von entweder T-Zell-Klonen oder T-Lymphozyten, wie sie in dem Kreislauf von Menschen vorhanden sind (PBMC), auszulösen;
e) Modifizieren der Aminosäuresequenz der Staphylokinase, deren Immunogenität reduziert werden soll, gemäß den T-Zell beseitigenden Modifikationen, die in der Aminosäuresequenz der Testpeptide gemacht wurden;
f) Herstellung und Reinigung der modifizierten Staphylokinase deren Immunogenität reduziert werden soll, in einem geeigneten Wirtsorganismus und Testen der biologischen Aktivität;
g) Wiederholen der Schritte c-f) mit den modifizierten Testpeptiden oder Staphylokinase, bis ein oder mehrere der T-Zell-Epitope, die ursprünglich darin enthalten sind, deutlich verringert oder beseitigt worden sind und Untersuchen der biologischen Aktivität.

4. Verfahren zum Verringern der Immunogenität von Staphylokinase, wobei das Verfahren die Schritte umfasst:
a) Entwurf einer Reihe von überlappenden Testpeptiden, von denen jedes eine Aminosäuresequenz besitzt, die einem Teil der Aminosäuresequenz der Staphylokinase deren Immunogenität reduziert werden soll, entspricht;
b) Identifizieren, welches der Testpeptide der Reihe ein oder mehr T-Zell-Epitope umfasst durch Test 1) und/oder 2) und 3) wie in Anspruch 1 definiert;
c) Modifizieren der Aminosäuresequenz von einem oder mehr der Testpeptide, die ein oder mehr T-Zell-Epitope enthalten;
d) Testen der modifizierten Peptide auf ihr Potential, T-Zellen zu aktivieren und die Proliferation von entweder T-Zell-Klonen und/oder T-Lymphozyten, wie sie in dem Kreislauf von Menschen vorhanden sind (PBMC), auszulösen;
e) Modifizieren der Aminosäuresequenz der Staphylokinase deren Immunogenität verringert werden soll gemäß den T-Zell beseitigenden Modifikationen, die in der Aminosäuresequenz der Testpeptide gemacht wurden;
f) Herstellung und Reinigung der modifizierten Staphylokinase deren Immunogenität reduziert werden soll in einem geeigneten Wirtsorganismus und Testen der biologischen Aktivität;
g) Wiederholen der Schritte c-f) mit den modifizierten Testpeptiden oder der Staphylokinase, bis ein oder mehrere T-Zell-Epitope, die ursprünglich darin enthalten sind, deutlich verringert oder beseitigt worden sind und Untersuchen der biologischen Aktivität.

5. Verfahren wie in den Ansprüchen 1-4 beansprucht, wobei die T-Zell-Epitope durch minimale Rest/Region Mutagenese beseitigt werden.

6. Verfahren wie in den Ansprüchen 1-5 beansprucht, wobei die T-Zell-Epitope durch Deletion oder Insertion von Resten in den/die identifizierten T-Zell-Epitopen beseitigt werden.

7. Verfahren wie in den Ansprüchen 1-6 beansprucht, wobei die T-Zell-Epitope durch Substitution mit menschlichen Sequenzen oder humanhomologen Sequenzen beseitigt werden.

8. Verfahren wie in den Ansprüchen 1-7 beansprucht, wobei die T-Zell-Epitope durch Erhöhen der Sensitivität der identifizierten T-Zell-Epitope für proteolytische endosomale Enzyme beseitigt werden.

9. Verfahren wie in den Ansprüchen 1-8 beansprucht, ferner umfassend die Identifizierung und Entfernung von HLA-DR verankernden Resten von Staphylokinase, um ihre Immunogenität zu verringern.

10. Verfahren wie in den Ansprüchen 1-9 beansprucht, wobei die biologische Aktivität von Staphylokinase mit verringerter Immunogenität nach Modifizierung der Aminosäuresequenz von Staphylokinase getestet wird.

11. Verfahren zur Herstellung einer Staphylokinase, die im Vergleich zur Wildtyp Staphylokinase eine verringerte Immunogenität besitzt, umfassend die folgenden Schritte:
a) Bereitstellen einer DNA-Sequenz, die mindestens die Aminosäuresequenz von Staphylokinase, die für ihre biologische Aktivität verantwortlich ist, kodiert;
b) Identifizieren von einem oder mehreren T-Zell-Epitopen, die in der Aminosäuresequenz der Wildtyp Staphylokinase enthalten sind durch:
1) Entwurf einer Reihe von überlappenden Testpeptiden, wobei jedes eine Aminosäuresequenz besitzt, die einem Teil der Aminosäuresequenz der Staphylokinase entspricht;
2) Identifizieren welches der Testpeptide der Reihe ein oder mehrere T-Zell-Epitope umfasst durch Test 1) und/oder 2) und 3), wie in Anspruch 1 definiert;
3) Modifizieren der Aminosäuresequenz von einem oder mehr der Testpeptide, die ein oder mehr T-Zell-Epitope enthalten;
4) Wiederholen von Schritt 2) und wahlweise Schritt 3) mit den modifizierten Testpeptiden bis ein oder mehr der T-Zell-Epitope, die ursprünglich darin enthalten sind, deutlich verringert oder beseitigt worden sind;
c) Modifizieren der DNA-Sequenz, um die Modifikationen der T-Zell-Epitope in der Aminosäuresequenz, die dadurch kodiert wird, einzuführen;
d) Einbringen der DNA-Sequenz in einen Wirtsorganismus;
e) Kultivieren des Wirtsorganismus unter Bedingungen, die die Expression der modifizierten Staphylokinase erlauben; und
f) Isolieren der Staphylokinase, die eine verringerte Immunogenität besitzt, aus dem Wirtsorganismus.

12. Verfahren zur Herstellung einer Staphylokinase, die im Vergleich zur Wildtyp Staphylokinase eine verringerte Immunogenität besitzt, umfassend die folgenden Schritte:
a) Bereitstellen einer DNA-Sequenz, die mindestens die Aminosäuresequenz von Staphylokinase kodiert, die für ihre biologische Aktivität verantwortlich ist;
b) Identifizieren und Modifizieren eines oder mehrerer T-Zell-Epitope, die in der Aminosäuresequenz der Wildtyp Staphylokinase enthalten sind durch:
1) Identifizieren welches der Testpeptide der Reihe ein oder mehr T-Zell-Epitope enthält durch Test 1) und/oder 2) und 3), wie in Anspruch 1) definiert;
2) Modifizieren der Aminosäuresequenz von einem oder mehr der Testpeptide, die ein oder mehrere T-Zell-Epitope enthalten;
3) Testen der modifizierten Peptide auf ihr Potential T-Zellen zu aktivieren und die Proliferation von entweder T-Zell-Klonen oder T-Lymphozyten, wie sie in dem Kreislauf von Menschen vorhanden sind (PBMC), auszulösen;
4) Modifizieren der Aminosäuresequenz der Staphylokinase deren Immunogenität verringert werden soll, gemäß den T-Zell beseitigenden Modifikationen, die in der Aminosäuresequenz der Testpeptide gemacht wurden;
5) Herstellung und Reinigung der modifizierten Staphylokinase deren Immunogenität verringert werden soll, in einem geeigneten Wirt und Testen der biologischen Aktivität;
6) Wiederholen der Schritte 2-5) mit der modifizierten Staphylokinase bis ein oder mehrere der T-Zell Epitope, die ursprünglich darin enthalten sind, deutlich verringert oder beseitigt worden sind und Untersuchen der biologischen Aktivität;
c) Modifizieren der DNA-Sequenz, um die Modifizierungen der T-Zell-Epitope in die Aminosäuresequenz, die dadurch kodiert wird, einzuführen;
d) Einbringen der DNA-Sequenz in einen Wirtsorganismus;
e) Kultivieren des Wirtsorganismus unter Bedingungen, die die Expression der modifizierten Staphylokinase erlauben; und
f) Isolieren der Staphylokinase, die eine verringerte Immunogenität besitzt, aus dem Wirtsorganismus.

13. Stapylokinasevarianten, in denen ein oder mehrere der folgenden immunogenen Regionen modifiziert sind, so dass die T-Zell-Epitope, die darin enthalten sind, beseitigt sind:
1-SSSFDKGKYKKGDDASY-17,
16-SYFEPTGPYLMVNVTGV-32,
56-TKEKIEYYVEWALDATA-72,
71-TAYKEFRVVELDPSAKI-87,
106-ITEKGFVVPDLSEHIKN-122, und
120-IKNPGFNLITKVVIEKK-136
mit der Ausnahme der Variante, die eine Kombination der folgenden Mutationen besitzt: K74A, E75A, R77A, E80A, D82A.

14. Staphylokinasevarianten, wie in Anspruch 13 beansprucht, mit im Vergleich zu Wildtyp Staphylokinase verringerter T-Zell-Reaktivität, die aber ihre biologische Aktivität beibehalten haben, wobei die Varianten die Aminosäuresequenz besitzen, die in Figur 1 dargestellt ist, mit einer oder mehreren der folgenden Aminosäuresubstitutionen in einer oder mehreren der folgenden immunogenen Regionen:
a) in der immunogenen Region, die die SakSTAR Reste 16-32 umfasst, die Substitutionen Y17L; F18L; F18E; E19D; P20Y, Y24A; P20Y, Y24S; G22S; G22P, P23G; oder N28S;
b) in der immunogenen Region, die die SakSTAR Reste 71-82 umfasst, die Substitutionen F76W, V79Y, D82A; R77A, E80A; R77S, E80S; R77A, E80A, D82A; K74Q, R77S, E80S; K74Q, R77S, E80S, D82G; K74Q, R77S, E80S, D82A; K74Q, R77S, E80A, D82A; K74Q, R77A, E80S, D82A; K74Q, R77S, E80S, D82S; oder V79Y, D82A;
c) in der immunogenen Region, die die SakSTAR Reste 106-122 umfasst, die Substitutionen V112T; V112S; D115N; E118S; H119A; H119S; V89L, L116Y; V89L, L116T; V112T, H119S; V112T, H119A; oder V112T, E118S, H119S;
d) in der immunogenen Region, die die SakSTAR Reste 120-136 umfasst, die Substitution K130Y.

15. Staphylokinasevarianten, wie in Anspruch 14 beansprucht, die eine Kombination von einer oder mehreren der Mutationen, die in Anspruch 14 aufgelistet sind, besitzen.

16. Staphylokinasevarianten, wie in Anspruch 15 beansprucht, die eine der folgenden Kombinationen von Mutationen besitzen
V112T, H119S, K130Y; R77E, E134R; V29L, L127V; K74Q, R77E, E80S, D82S, E134R; R77S, E80S, V112T, H119S; R77S, E80S, V112T, H119S, K130Y; R77A, E80A, V112T, H119S; R77A, E80A, V112T, H119S, K130Y; K74Q, R77S, E80S, V112T, H119S; K74Q, R77S, E80S, V112T, H119S, K130Y; K74Q, R77S, E80S, D82S, V112T, H119S; oder K74Q, R77S, E80S; D82S, V112T, H119S, K130Y.

17. Staphylokinasevarianten, ausgewählt aus der Gruppe bestehend aus
SakSTAR (P20Y, Y24A), SakSTAR (P20Y, Y24S), SakSTAR (E19D), SakSTAR (Y17L), SakSTAR (F18L), SakSTAR (F18E), SakSTAR (G22S), SakSTAR (G22S, P23G), SakSTAR (N28S), SakSTAR (V29L, L127V), SakSTAR (R77E, E134R), SakSTAR (K74Q, R77E, E80S, D82S, E134R), SakSTAR (K74Q, R77E, E80S, D82S, V112T, H119S, E134R), SakSTAR (K74Q, R77E, E80S, D82S, V112T, H119S, E134R, K130Y), SakSTAR (R77S, E80S, V112T, H119S), SakSTAR (R77S, E80S, V112T, H119S, K130Y), SakSTAR (R77A, E80A, V112T, H119S), SakSTAR (R77A, E80A, V112T, H119S, K130Y), SakSTAR (K74Q, R77S, E80S, V112T, H119S), SakSTAR (K74Q, R77S, E80S, V112T, H119S, K130Y), SakSTAR (K74Q, R77S, E80S, D82S, V112T, H119S), SakSTAR (K74Q, R77S, E80S, D82S, V112T, H119S, K130Y), SakSTAR (V112T, H119A), SakSTAR (V112T, H119S), SakSTAR (V112T, E118S, H119S), SakSTAR (V112T, H119S, K130Y), SakSTAR (V112T), SakSTAR (V112S), SakSTAR (H119A), SakSTAR (H119S), SakSTAR (V89L, L116Y), SakSTAR (V89L, L116T), SakSTAR (V112T), SakSTAR (V112S), SakSTAR (D115N), SakSTAR (E118S), SakSTAR (K130Y).

18. Staphylokinasevarianten wie in den Ansprüchen 13-17 beansprucht für die Verwendung bei der Behandlung, Diagnose oder Prophylaxe.

19. Verwendung einer Staphylokinasevariante, wie in den Ansprüchen 13-17 beansprucht, für die Herstellung einer pharmazeutischen Zusammensetzung für die Behandlung, Diagnose oder Prophylaxe eines Menschen.

20. Pharmazeutische Zusammensetzung umfassend eine Staphylokinasevariante, wie in den Ansprüchen 13-17 beansprucht, zusammen mit einem pharmazeutisch annehmbaren Träger, Verdünnungsmittel oder Hilfsstoff.

## Revendications

1. Procédé de réduction de l'immunogénicité de la staphylokinase, lequel procédé comprend les étapes consistant à :
a) concevoir une série de peptides tests chevauchants ayant chacun une séquence d'acides aminés qui correspond à une partie de la séquence d'acides aminés de la staphylokinase ;
b) identifier lequel des peptides tests de la série comprend un ou plusieurs épitopes de lymphocyte T ;
c) modifier la séquence d'acides aminés d'un ou plusieurs des peptides tests comprenant un ou plusieurs épitopes de lymphocyte T ;
d) répéter l'étape b) et éventuellement l'étape c) avec les peptides tests modifiés jusqu'à ce qu'un ou plusieurs des épitopes de lymphocyte T compris à l'origine dans les peptides tests soient en quantité réduite ou soient éliminés ;
e) modifier la séquence d'acides aminés de la staphylokinase selon les modifications pour l'élimination des lymphocytes T effectuées dans la séquence d'acides aminés des peptides tests pour produire une staphylokinase modifiée,
**caractérisé en ce que**
l'identification des peptides tests comprenant un épitope de lymphocyte T est effectuée par une combinaison des tests suivants :
1) un dosage des lymphocytes T fonctionnel dans lequel la prolifération d'un ou plusieurs clones de lymphocyte T après stimulation du ou des clones de lymphocyte T avec un des peptides tests signifie que ledit peptide test comprend un épitope de lymphocyte T potentiel ; et/ou
2) un dosage de lymphocyte T fonctionnel dans lequel la prolifération d'un ou plusieurs lymphocytes T tels qu'ils sont présents dans la circulation des êtres humains (cellules mononuclées du sang périphérique, PBMC) après stimulation des PBMC avec un des peptides tests signifie que ledit peptide test comprend un épitope de lymphocyte T ; et
3) détermination de l'énergie d'interaction dudit peptide test avec le sillon de liaison d'un ou plusieurs haplotypes de HLA-DR au moyen d'une modélisation par ordinateur, dans laquelle le peptide test est identifié comme comprenant un épitope de lymphocyte T potentiel quand, selon son énergie d'interaction, il s'adapte dans le sillon de liaison de HLA-DR,
dans lequel un peptide test est considéré comme comprenant un épitope de lymphocyte T s'il est identifié dans le test 1) et/ou 2) et 3).

2. Procédé selon la revendication 1, comprenant en outre une ou plusieurs répétitions des étapes b) à e) avec la staphylokinase modifiée à la place des peptides tests.

3. Procédé selon la revendication 1 ou 2, lequel procédé comprend les étapes consistant à :
a) concevoir une série de peptides tests chevauchants ayant chacun une séquence d'acides aminés qui correspond à une partie de la séquence d'acides aminés de la staphylokinase dont l'immunogénicité doit être réduite ;
b) identifier lequel des peptides tests de la série comprend un ou plusieurs épitopes de lymphocyte T au moyen d'un test 1) et/ou 2) et 3) tel que défini dans la revendication 1 ;
c) modifier la séquence d'acides aminés dans un ou plusieurs des peptides tests comprenant un ou plusieurs épitopes de lymphocyte T ;
d) tester les peptides modifiés pour leur potentiel à activer les lymphocytes T et à induire une prolifération soit des clones de lymphocyte T soit des lymphocytes T tels qu'ils sont présents dans la circulation des êtres humains (PBMC) ;
e) modifier la séquence d'acides aminés de la staphylokinase dont l'immunogénicité doit être réduite selon les modifications pour l'élimination des lymphocytes T effectuées dans la séquence d'acides aminés des peptides tests ;
f) construire et purifier la staphylokinase modifiée dont l'immunogénicité doit être réduite dans un hôte approprié, et tester son activité biologique ;
g) répéter les étapes c à f) avec les peptides tests ou la staphylokinase modifiés jusqu'à ce qu'un ou plusieurs des épitopes de lymphocyte T compris à l'origine dans ceux-ci soient en quantité significativement réduite ou soient éliminés et prendre en compte l'activité biologique.

4. Procédé de réduction de l'immunogénicité de la staphylokinase, lequel procédé comprend les étapes consistant à :
a) concevoir une série de peptides tests chevauchants ayant chacun une séquence d'acides aminés qui correspond à une partie de la séquence d'acides aminés de la staphylokinase dont l'immunogénicité doit être réduite ;
b) identifier lequel des peptides tests de la série comprend un ou plusieurs épitopes de lymphocyte T au moyen d'un test 1) et/ou 2) et 3) tel que défini dans la revendication 1 ;
c) modifier la séquence d'acides aminés d'un ou plusieurs des peptides tests comprenant un ou plusieurs épitopes de lymphocyte T ;
d) tester les peptides modifiés pour leur potentiel à activer les lymphocytes T et à induire une prolifération soit des clones de lymphocyte T et/ou soit de lymphocytes T tels qu'ils sont présents dans la circulation des êtres humains (PBMC) ;
e) modifier la séquence d'acides aminés de la staphylokinase dont l'immunogénicité doit être réduite selon les modifications pour l'élimination des lymphocytes T effectuées dans la séquence d'acides aminés des peptides tests ;
f) construire et purifier la staphylokinase modifiée dont l'immunogénicité doit être réduite dans un hôte approprié, et tester son activité biologique ;
g) répéter les étapes c à f) avec les peptides tests ou la staphylokinase modifiés jusqu'à ce qu'un ou plusieurs des épitopes de lymphocyte T compris à l'origine dans ceux-ci soient en quantité significativement réduite ou soient éliminés et prendre en compte l'activité biologique.

5. Procédé selon les revendications 1 à 4, dans lequel les épitopes de lymphocyte T sont éliminés par mutagenèse d'un résidu/d'une région minime.

6. Procédé selon les revendications 1 à 5, dans lequel les épitopes de lymphocyte T sont éliminés par délétion ou insertion de résidus dans les épitopes de lymphocyte T identifiés.

7. Procédé selon les revendications 1 à 6, dans lequel les épitopes de lymphocyte T sont éliminés par substitution avec des séquences humaines, ou des séquences homologues humaines.

8. Procédé selon les revendications 1 à 7, dans lequel les épitopes de lymphocyte T sont éliminés par augmentation de la sensibilité des épitopes de lymphocyte T identifiés aux enzymes endosomales protéolytiques.

9. Procédé selon les revendications 1 à 8, comprenant en outre l'identification et le retrait de résidus d'ancrage de HLA-DR de la staphylokinase pour réduire son immunogénicité.

10. Procédé selon les revendications 1 à 9, dans lequel l'activité biologique de la staphylokinase avec une immunogénicité réduite est testée après modification de la séquence d'acides aminés de la staphylokinase.

11. Procédé de production d'une staphylokinase ayant une immunogénicité réduite en comparaison avec la staphylokinase de type sauvage, comprenant les étapes suivantes :
a) apporter une séquence d'ADN codant au moins la séquence d'acides aminés de la staphylokinase qui supporte son activité biologique ;
b) identifier un ou plusieurs des épitopes de lymphocyte T compris dans la séquence d'acides aminés de la staphylokinase de type sauvage par :
1) conception d'une série de peptides tests chevauchants ayant chacun une séquence d'acides aminés qui correspond à une partie de la séquence d'acides aminés de la staphylokinase ;
2) identification du peptide test de la série qui comprend un ou plusieurs épitopes de lymphocyte T au moyen du test 1) et/ou 2) et 3) tel que défini dans la revendication 1 ;
3) modification de la séquence d'acides aminés d'un ou plusieurs des peptides tests comprenant un ou plusieurs épitopes de lymphocyte T ;
4) répétition de l'étape 2) et éventuellement de l'étape 3) avec les peptides tests modifiés jusqu'à ce qu'un ou plusieurs des épitopes de lymphocyte T à l'origine compris dans ceux-ci soient en une quantité significativement réduite ou soient éliminés ;
c) modifier la séquence d'ADN pour introduire les modifications à l'épitope ou aux épitopes de lymphocyte T dans la séquence d'acides aminés codée ainsi ;
d) introduire la séquence d'ADN dans un organisme hôte ;
e) cultiver l'organisme hôte dans des conditions permettant l'expression de la staphylokinase modifiée ; et
f) isoler la staphylokinase ayant une immunogénicité réduite de l'organisme hôte.

12. Procédé de production d'une staphylokinase ayant une immunogénicité réduite en comparaison avec la staphylokinase de type sauvage, comprenant les étapes suivantes :
a) apporter d'une séquence d'ADN codant au moins la séquence d'acides aminés de la staphylokinase qui supporte son activité biologique ;
b) identifier et modifier un ou plusieurs des épitopes de lymphocyte T compris dans la séquence d'acides aminés de la staphylokinase de type sauvage par :
1) identification des peptides tests de la série qui comprennent un ou plusieurs épitopes de lymphocyte T au moyen d'un test 1) et/ou 2) et 3) tel que défini dans la revendication 1 ;
2) modification de la séquence d'acides aminés d'un ou plusieurs des peptides tests comprenant un ou plusieurs épitopes de lymphocyte T ;
3) test des peptides modifiés pour leur potentiel à activer les lymphocytes T et à induire une prolifération soit des clones de lymphocyte T soit des lymphocytes T tels qu'il sont présents dans la circulation des êtres humains (PBMC) ;
4) modification de la séquence d'acides aminés de la staphylokinase dont l'immunogénicité doit être réduite selon les modifications pour l'élimination des lymphocytes T effectuées dans la séquence d'acides aminés des peptides tests ;
5) construction et purification de la staphylokinase modifiée dont l'immunogénicité doit être réduite dans un hôte approprié, et test de son activité biologique ;
6) répétition des étapes 2 à 5) avec la staphylokinase modifiée jusqu'à ce qu'un ou plusieurs des épitopes de lymphocyte T compris à l'origine dans celle-ci soient en quantité significativement réduite ou soient éliminés et prendre en compte l'activité biologique ;
c) modifier la séquence d'ADN pour introduire les modifications à l'épitope ou aux épitopes de lymphocyte T dans la séquence d'acides aminés codée ainsi ;
d) introduire la séquence d'ADN dans un organisme hôte ;
e) cultiver l'organisme hôte dans des conditions permettant l'expression de la staphylokinase modifiée ; et
f) isoler la staphylokinase ayant une immunogénicité réduite de l'organisme hôte.

13. Variants de la staphylokinase dans lesquels une ou plusieurs des régions immunogènes suivantes sont modifiées de sorte que les épitopes de lymphocyte T contenus dans celle-ci soient éliminés :
1-SSSFDKGKYKKGDDASY-17,
16-SYFEPTGPYLMVNVTGV-32,
56-TKEKIEYYVEWALDATA-72,
71-TAYKEFRWELDPSAKI-87,
106-ITEKGFVVPDLSEHIKN-122, et
120-IKNPGFNLITKVVIEKK-136
à l'exclusion du variant ayant la combinaison des mutations suivantes : K74A, E75A, R77A, E80A, D82A.

14. Variants de la staphylokinase selon la revendication 13 avec une réactivité envers les lymphocytes T réduite en comparaison avec la staphylokinase de type sauvage mais conservation de son activité biologique, lesquels variants ont la séquence d'acides aminés décrite dans la figure 1 avec une ou plusieurs des substitutions d'acides aminés suivantes dans une ou plusieurs des régions immunogènes suivantes :
a) dans la région immunogène comprenant les résidus 16 à 32 de SakSTAR, les substitutions Y17L; F18L ; F18E ; E19D ; P20Y, Y24A ; P20Y, Y24S ; G22S ; G22P, P23G ; ou N28S ;
b) dans une région immunogène comprenant les résidus 71 à 82 de SakSTAR, les substitutions F76W, V79Y, D82A ; R77A, E80A ; R77S, E80S ; R77A, E80A, D82A ; K74Q, R77S, E80S ; K74Q, R77S, E80S, D82G ; K74Q, R77S, E80S, D82A ; K74Q, R77S, E80A, D82A ; K74Q, R77A, E80S, D82A ; K74Q, R77S, E80S, D82S ; ou V79Y, D82A ;
c) dans une région immunogène comprenant les résidus 106 à 122 de SakSTAR, les substitutions V112T ; V112S; D115N ; E118S ; H119A ; H119S ; V89L, L116Y ; V89L, L116T ; V112T, H119S ; V112T, H119A ; ou V112T, E118S, H119S;
d) dans une région immunogène comprenant les résidus 120 à 136 de SakSTAR, la substitution K130Y.

15. Variants de la staphylokinase selon la revendication 14, ayant une combinaison d'une ou plusieurs des mutations listées dans la revendication 14.

16. Variants de la staphylokinase selon la revendication 15, ayant une des combinaisons de mutations suivantes
V112T, H119S, K130Y; R77E, E134R; V29L, L127V ; K74Q, R77E, E80S, D82S, E134R ; R77S, E80S, V112T, H119S ; R77S, E80S, V112T, H119S, K130Y ; R77A, E80A, V112T, H119S ; R77A, E80A, V112T, H119S, K130Y ; K74Q, R77S, E80S, V112T, H119S ; K74Q, R77S, E80S, V112T, H119S, K130Y ; K74Q, R77S, E80S, D82S, V112T, H119S ; ou K74Q, R77S, E80S, D82S, V112T, H119S, K130Y.

17. Variant de la staphylokinase choisi dans le groupe constitué par SakSTAR(P20Y, Y24A), SakSTAR(P20Y, Y24S), SakSTAR(E19D), SakSTAR(Y17L), SakSTAR(F18L), SakSTAR(F18E), SakSTAR(G22S), SakSTAR(G22S, P23G), SakSTAR(N28S), SakSTAR(V29L, L127V), SakSTAR(R77E, E134R), SakSTAR(K74Q, R77E, E80S, D82S, E134R), SakSTAR(K74Q, R77E, E80S, D82S, V112T, H119S, E134R), SakSTAR(K74Q, R77E, E80S, D82S, V112T, H119S, E134R, K130Y), SakSTAR(R77S, E80S, V112T, H119S), SakSTAR(R77S, E80S, V112T, H119S, K130Y), SakSTAR(R77A, E80A, V112T, H119S), SakSTAR(R77A, E80A, V112T, H119S, K130Y), SakSTAR(K74Q, R77S, E80S, V112T, H119S), SakSTAR(K74Q, R77S, E80S, V112T, H119S, K130Y), SakSTAR(K74Q, R77S, E80S, D82S, V112T, H119S), SakSTAR(K74Q, R77S, E80S, D82S, V112T, H119S, K130Y), SakSTAR(V112T, H119A), SakSTAR(V112T, H119S), SakSTAR(V112T, E118S, H119S), SakSTAR(V112T, H119S, K130Y), SakSTAR(V112T), SakSTAR(V112S), SakSTAR(H119A), SakSTAR(H119S), SakSTAR(V89L, L116Y), SakSTAR(V89L, L116T), SakSTAR(V112T), SakSTAR(V112S), SakSTAR(D115N), SakSTAR(E118S), SakSTAR(K130Y).

18. Variant de la staphylokinase selon les revendications 13 à 17 pour une utilisation dans le traitement, le diagnostic ou la prophylaxie.

19. Utilisation d'un variant de la staphylokinase selon les revendications 13 à 17, pour la préparation d'une composition pharmaceutique pour le traitement, le diagnostic ou la prophylaxie d'un sujet humain.

20. Composition pharmaceutique comprenant un variant de la staphylokinase selon les revendications 13 à 17, conjointement avec un support, des diluants ou un excipient acceptables d'un point de vue pharmaceutique.
